# EUROPEAN PATENT SPECIFICATION

(11) **EP 3 562 523 B1**
(45) Date of publication and mention of the grant of the patent: **02.12.2026**
(21) Application number: 17892151.6
(22) Date of filing: 22.12.2017
(51) Int. Cl.: A61L 27/14, A61L 27/54, A61L 27/56

(54) **METHODS AND SYSTEMS FOR TREATING A SITE OF A MEDICAL IMPLANT**
VERFAHREN UND SYSTEME ZUR BEHANDLUNG EINER STELLE EINES MEDIZINISCHEN IMPLANTATS
PROCÉDÉS ET SYSTÈMES DE TRAITEMENT D'UN SITE D'IMPLANT MÉDICAL

(30) Priority: 29.12.2016 US 201662440370 P
(43) Date of publication of application: 06.11.2019
(73) Proprietor: Tempo Therapeutics, Inc., San Diego, California 92109 (US)
(72) Inventor: WEAVER, Westbrook, San Diego, California 92109 (US); DESHAYES, Stephanie, San Diego, California 92109 (US); TIMKO, Samuel, San Diego, California 92109 (US)
(74) Representative: HGF
(86) International application number: PCT/US2017/068243
(87) International publication number: WO 2018/136205

(56) References cited:
- WO-A1-2014/025312
- WO-A1-2017/136427
- WO-A1-2017/142879
- US-A1- 2009 011 009
- US-A1- 2011 257 623
- US-A1- 2011 257 623
- US-A1- 2012 116 511
- US-A1- 2016 279 283
- US-A1- 2016 279 283
- US-A1- 2016 303 281

## Description

### BACKGROUND OF THE DISCLOSURE

Current porous synthetic hydrogels used as such healing agents are produced by methods that require toxic removal of porogens to form pores, or degradation of encapsulated microparticles, which requires these constructs to be either cast *ex vivo,* preventing them from seamlessly integrating with the surrounding tissue like an injectable biomaterial or requires long-term *in vivo* development to resolve the porous structure.
US 2016/279283 A1 discloses a biomaterial that promotes rapid regeneration of tissue while maintaining structural support of surrounding tissue of a wound.
US 2011/257623 A1 discloses an implantable device coated with microporous surface layers (hydrogel) that improve bio-integration at the interface of the implantable device and the surrounding tissue.

### SUMMARY OF THE DISCLOSURE

The scope of protection is defined by the claims. Any references in the description to methods of treatment refer to the products of the present invention for use in a method for treatment.

Cell migration to a site of injury or surgery is essential for healing. Therefore, wound healing agents used at these sites ideally do not impede cellular migration. Implantation of medical devices, such as biomaterials, prosthetics and cardiac pacemakers, is common practice in modern medicine. However, tissues that are subjected to medical device implantation produce a complex set of immune responses, including for example, to the device and the implantation procedure, including inflammation, wound healing, foreign body reactions and fibrous encapsulation of the device. These responses do not always result in a desirable outcome for the patient. For instance, the site of implantation may develop scar tissue or fibrotic tissue that is deleterious to the function of the surrounding tissue and the subject.

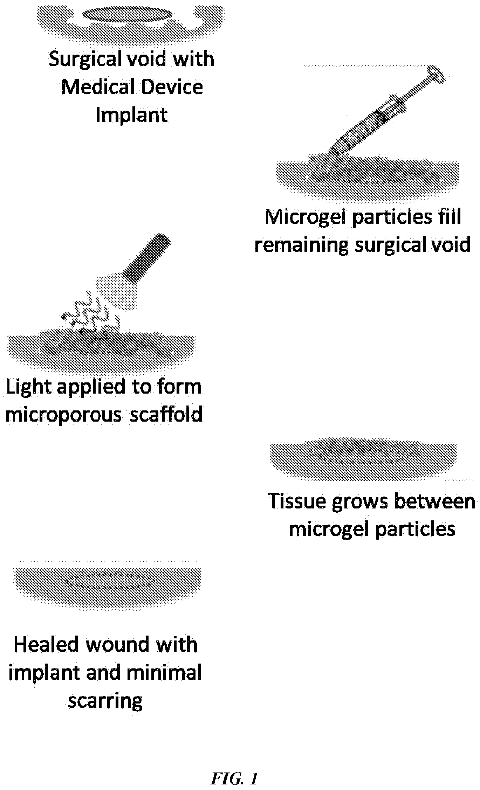

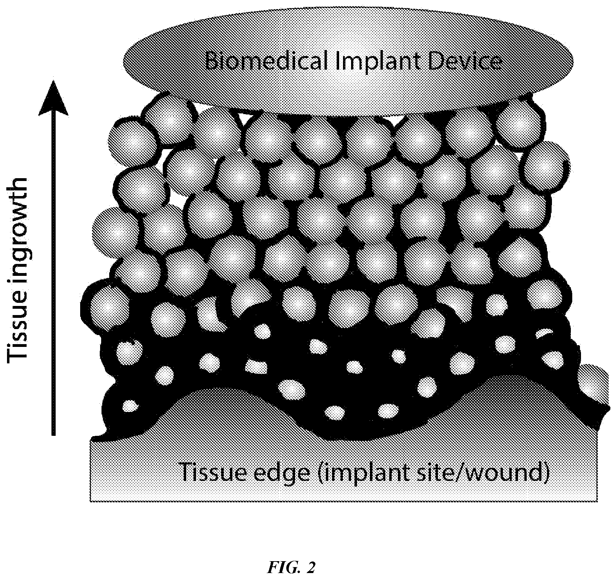

The systems disclosed herein aim to improve the tissue-device interface through the use of microporous gel systems. These microporous gel systems
are applied to a surgical void, such as a medical device implantation site, and around the medical device. A stimulus such as light is then applied to the microporous gel system to create a microporous scaffold (see *e.g.,* FIG. 1). The microporous gel system disclosed herein can act as a buffer between the tissue and the device, promoting healing of the tissue and incorporation of the device into the tissue, while mitigating or avoiding fibrous encapsulation of the device, inflammation or infection. The presence of the interconnected pores between the medical implant and the surrounding tissue (see, *e.g.,* FIG. 2), provided by the microporous gel system, create a unique environment that does not lead to a chronic inflammatory response or fibrous tissue formation. The ability of tissue (or cells thereof) to migrate into the material without the need for degradation is an important aspect to the invention in the context of implanted medical devices.

In examples not claimed, microporous gel systems disclosed herein provide for prevention and treatment of infections via antimicrobial activity. In examples not claimed , microporous gel systems disclosed herein provide for mitigation of other negative characteristics of surgical implant sites such as pain and chronic inflammation. In examples not claimed , microporous gel systems disclosed herein provide for stable shelf products that release a tissue site treatment *(e.g.,* an antimicrobial treatment) when placed in a surgical/implant site. Tissue site treatments may provide for minimal/absent fibrosis around a surgical site pocket via anti-fibrotic capability of microporous scaffold. Tissue site treatments may provide for minimal/absent inflammation at a surgical site pocket via anti-inflammatory capability of microporous scaffolds.

In some instances, microporous gel systems disclosed herein provide for physically stabilizing medical devices in an implant or surgical site. In some instances, microporous gel systems disclosed herein provide for holding a medical device in place by a microporous scaffold. In some instances, medical device of one size can be applied to surgical/implant sites of different shapes and sizes, with extra space in the surgical/implant site and around the medical device filled by a microporous gel system disclosed herein during/after implantation. Using a microporous gel system disclosed herein, medical devices and implants of many sizes and shapes can be interfaced with surgical pockets (in a tissue) of varying sizes and shapes because excess surgical site space is filled by the microporous gel system.

Features and characteristics of microporous gel systems disclosed herein provide for applying the microporous gel systems in a manner that is custom to a subject and the features of the subject's surgical site or implant site.

As one of skill in the art will understand from the description and examples presented herein, medical device manufacturing (size, shape, etc.) is not dependent upon manufacturing of microporous scaffolds disclosed herein, or vice versa. Advantageously, the adaptable, customizable microporous scaffolds disclosed herein may be applied immediately to medical devices of any shape, size, etc., and/or surgical pockets of any shape, size, etc.

According to the invention, there is provided a microporous gel system according to claim 1.

The first annealing component and the second annealing component may be the same. The first annealing component and the second annealing component may be different. The at least one annealing component may be a substrate for an enzyme of a mammalian subject. A first annealing component and a second annealing component are linked together when exposed to a condition in a mammalian subject. The medical device is a medical implant. The medical device may comprise an electrode. The medical device may comprise an electrical component. The medical device may comprise a coating, wherein the coating comprises at least one of the annealing component and an annealing agent. The medical implant may be a cardiac implantable electronic device. The cardiac implantable electronic device may be a pacemaker. The cardiac implantable electronic device may be a defibrillator. The medical implant may be a neural implantable electronic device. The stabilized scaffold may maintain placement of the medical device in a surgical void of a subject.

In some instances, the stabilized scaffold comprises non-covalent bonds between the flowable microgel particles. In some instances, the stabilized scaffold comprises covalent bonds between the flowable microgel particles. In some instances, systems comprise a therapeutic agent. In some instances, the therapeutic agent is an anti-inflammatory agent, an antimicrobial agent, or an analgesic. In some instances, the therapeutic agent is incorporated in the stabilized scaffold. In some instances, systems comprise a therapeutic agent, wherein the stabilized scaffold releases the therapeutic agent from the stabilized scaffold when the stabilized scaffold is present in a mammalian subject. In some instances, the stabilized scaffold releases at least a portion of the therapeutic agent from the stabilized scaffold in less than one day from its initial presence in the mammalian subject. In some instances, the stabilized scaffold releases the therapeutic agent from the stabilized scaffold over a period of less than 1 day to 100 days. In some instances, systems comprise a therapeutic agent releasing agent that releases the therapeutic agent from the stabilized scaffold. In some instances, the therapeutic agent is released by tissue mediated hydrolysis. In some instances, the therapeutic agent is released by passive hydrolysis. In some instances, the therapeutic agent is released by a temperature change. In some instances, systems comprise a nanoparticle. In some instances, the therapeutic agent is connected to or contained within the nanoparticle. In some instances, the nanoparticle is a mesoporous silica nanoparticle. In some instances, the nanoparticle comprises poly(lactic-co-glycolic acid). In some instances, the nanoparticle comprises chitosan. In some instances, the nanoparticle comprises hyaluronic acid. In some instances, the nanoparticle comprises a poly(anhydride), a poly(amide), a poly(ortho ester), a polycaprolactone, or a combination thereof. In some instances, the nanoparticle comprises a polymer with a lower critical solution temperature (LCST). In some instances, the polymer is poly(N-isopropylacrylamide) or a copolymer thereof. In some instances, the nanoparticle comprises a polymer with an upper critical solution temperature (UCST). In some instances, the polymer is poly(hydroxyethylmethacrylate), polyethylene oxide, or poly(ethyleneoxide)-poly(propyleneoxide)-poly(ethyleneoxide). In some instances, the nanoparticle comprises a self-immolating polymer. In some instances, the polymer is poly(p-aminobenzyl oxycarbonyl). In some instances, the polymer is capped with a cage that can be released upon a stimulus. In some instances, the system comprises a core-shell nanoparticle system.

In some instances, systems comprise an application device, wherein the application device is configured to apply the flowable microgel particles and the at least one annealing component to a tissue of a subject. In some instances, the application device comprises a syringe, a spatula, a squeezable tube or a cannula.

In some instances, the microporous gel system has a shelf life of at least about one year at room temperature.

Disclosed herein, but not claimed , are methods of treating a site of a medical device in a tissue of a subject comprising administering to the site: a collection of flowable microgel particles, wherein the flowable microgel particles comprise a backbone polymer; at least one annealing component; and a medical device, wherein the flowable microgel particles are capable of being linked together via the at least one annealing component to form a stabilized scaffold having interstitial spaces therein.

Disclosed herein, but not claimed ,are methods of reducing or preventing fibrosis at a site of a medical device in a tissue of a subject comprising administering to the site: a collection of flowable microgel particles, wherein the flowable microgel particles comprise a backbone polymer; at least one annealing component; and a medical device, wherein the flowable microgel particles are capable of being linked together via the at least one annealing component to form a stabilized scaffold having interstitial spaces therein.

Disclosed herein, but not claimed, are methods of reducing or preventing inflammation at a site of a medical device in a tissue of a subject comprising administering to the site: a collection of flowable microgel particles, wherein the flowable microgel particles comprise a backbone polymer; at least one annealing component; and a medical device, wherein the flowable microgel particles are capable of being linked together via the at least one annealing component to form a stabilized scaffold having interstitial spaces therein. In some instances, the medical device is a surgical device. In some instances, the medical device is a medical implant. In some instances, methods comprise administering at least one of the annealing component and the flowable microgel particles to the site before administering the medical device to the site. In some instances, methods comprise administering at least one of the annealing component and the flowable microgel particles to the site after administering the medical device to the site. In some instances, methods comprise co-administering at least one of the annealing component and the flowable microgel particles, and the medical device to the site. In some instances, methods comprise administering at least one of the annealing component and the flowable microgel particles with a syringe, cannula, squeezable tube or spatula. In some instances, methods comprise administering an annealing agent. In some instances, methods comprise administering the annealing agent before administering at least one of the annealing component and the flowable microgel particles. In some instances, methods comprise administering the annealing agent after administering at least one of the annealing component and the flowable microgel particles. In some instances, methods comprise co-administering the annealing agent and at least one of the annealing component and the flowable microgel particles. In some instances, methods comprise administering a therapeutic agent to the site. In some instances, methods comprise administering a therapeutic agent releasing agent to the site, wherein the therapeutic agent releasing agent releases the therapeutic agent from the stabilized scaffold to the site or tissue. In some instances, methods comprise incorporating the therapeutic agent into the stabilized scaffold. In some instances, the stabilized scaffold comprises a core-shell nanoparticle system wherein the therapeutic agent is connected to or contained within the core-shell nanoparticle system, comprising applying an external stimulus to the stabilized scaffold to release the therapeutic agent to the site or tissue. In some instances, the external stimulus selected from light, electromagnetic radiation, or temperature change. In some instances, methods comprise changing a condition of the site after formation of the stabilized scaffold. In some instances, methods comprise changing a condition of the site before formation of the stabilized scaffold. In some instances, changing the condition comprises at least one of changing temperature of the site, changing pH of the site, changing chemistry of the site, applying an exogenous enzyme, activating an endogenous enzyme, applying a magnetic field, applying a form of radiation, applying light, and applying ultrasound.

Disclosed herein, but not claimed , are methods of treating a heart condition comprising administering to a subject in need thereof: a collection of flowable microgel particles, wherein the flowable microgel particles comprise a backbone polymer; at least one annealing component; and a cardiac implantable electronic device, wherein the flowable microgel particles are capable of being linked together via the at least one annealing component to form a stabilized scaffold having interstitial spaces therein. In some instances, the heart condition is a heart arrhythmia. In some instances, the heart condition is a sustained ventricular tachycardia. In some instances, the heart condition is a ventricular fibrillation.

Disclosed herein, but not claimed , are methods of treating a neurological condition comprising administering to a subject in need thereof: a collection of flowable microgel particles, wherein the flowable microgel particles comprise a backbone polymer; at least one annealing component; and a neural implantable electronic device, wherein the flowable microgel particles are capable of being linked together via the at least one annealing component to form a stabilized scaffold having interstitial spaces therein.

Disclosed herein, but not claimed , are methods of producing a microporous scaffold, comprising: synthesizing a first portion of flowable microgel particle in the presence of a first annealing component and a second annealing component, wherein there is more of the first annealing component than the second annealing component to produce a first functionalized microgel particle; synthesizing a second portion of flowable microgel particle in the presence of the first annealing component and the second annealing component, wherein there is more of the second annealing component than the first annealing component to produce a second functionalized microgel particle; combining the first functionalized microgel particle and the second functionalized microgel particle such that the first functionalized microgel particle and the second functionalized microgel particle connect, thereby producing a microporous scaffold of microgel particles having interstitial spaces therebetween. In some instances, there is at least 1% more of the first annealing component than the second annealing component in step (a). In some instances, there is at least 1% more of the second annealing component than the first annealing component in step (b). In some instances, at least one of the first annealing component and the second annealing component comprise a functional group selected from a vinyl sulfone, thiol, amine, imidazole, aldehyde, ketone, hydroxyl, azide, alkyne, vinyl, alkene, maleimide, carboxyl, N-hydroxysuccinimide (NHS) ester, isocyanate, isothiocyanate, hydroxylamine, and thione. In some instances, the first functionalized microgel particle and the second functionalized microgel particle connect through a reaction selected from Michael addition, amide bond coupling, Diels-Alder cycloaddition, Huisgen 1,3-dipolar cycloaddition, reductive amination, carbamate linkage, ester linkage, thioether linkage, disulfide bonding, hydrazone bonding, oxime coupling, and thiourea coupling. In some instances, the first functionalized microgel particle and the second functionalized microgel particle connect to produce a covalent bond. In some instances, the first functionalized microgel particle and the second functionalized microgel particle connect to produce a non-covalent bond. In some instances, the first functionalized microgel particle and the second functionalized microgel particle connect to produce a connection selected from a C-C bond, an amide bond, an amine bond, a carbamate linkage, an ester linkage, a thioether linkage, a disulfide bond, a hydrazine bond, an oxime coupling and a thiourea coupling. In some instances, at least one step of the method is performed *in situ.*

Disclosed herein, but not claimed , are methods of producing a microporous scaffold, comprising: synthesizing flowable microgel particles; contacting a first portion of the flowable microgel particles with a first annealing component to produce a first functionalized microgel particle; contacting a second portion of the flowable microgel particles with a second annealing component to produce a second functionalized microgel particle; combining the first functionalized microgel particle and the second functionalized microgel particle such that the first functionalized microgel particle and the second functionalized microgel particle connect, thereby producing a microporous scaffold of microgel particles having interstitial spaces therebetween. In some instances, at least one of the first annealing component and the second annealing component comprise a reactive moiety selected from a catechol, a sialic acid, a boronic acid, a molecular cage, adamantane, biotin, and streptavidin. In some instances, the molecular cage is selected from a cyclodextrin, a cucurbituril, a calixarene, a pillararene, a crown ether, a cavitand, a cryptand, and a carcerand. In some instances, the first functionalized microgel particle and the second functionalized microgel particle connect through a covalent bond. In some instances, the covalent bond is selected from an amide, ester, C-C bond, carbamate, disulfide bond, oxime, thiourea, hydrazone, and imine. In some instances, the first functionalized microgel particle and the second functionalized microgel particle connect through a non-covalent bond. In some instances, the non-covalent bond is selected from an electrostatic interaction, a hydrogen bond, a cation-π, π-π stack, a metal-ligand bond, a van der Waals interaction, and a non-covalent host-guest inclusion complex. In some instances, at least one step of the method is performed *in situ.* In some instances, methods comprise contacting the first functionalized microgel particle and the second functionalized microgel particle with an intercrosslinker in order to connect the first functionalized microgel particle and the second functionalized microgel particle. In some instances, contacting occurs *in situ.* In some instances, contacting occurs after synthesizing the flowable microgel particles. In some instances, the intercrosslinker comprises at least one functional group. In some instances, the intercrosslinker comprises at least two functional groups. In some instances, at least one functional group is selected from a vinyl sulfone, a thiol, an amine, an imidazole, an aldehyde, a ketone, a hydroxyl, an azide, an alkyne, a vinyl, an alkene, a maleimide, a carboxyl, a N-Hydroxysuccinimide (NHS) ester, an isocyanate, an isothiocyanate, ahydroxylamine, and a thione. In some instances, connecting the first functionalized microgel particle and the second functionalized microgel particle comprises a reaction selected from Michael addition, amide bond coupling, Diels-Alder cycloaddition, Huisgen 1,3-dipolar cycloaddition, reductive amination, carbamate linkage, ester linkage, thioether linkage, disulfide bond, hydrazone bond, oxime coupling, and thiourea coupling. In some instances, methods comprise contacting the first functionalized microgel particle and the second functionalized microgel particle with an intercrosslinking agent. In some instances, the intercrosslinking agent comprises a reducing agent. In some instances, the reducing agent comprises at least one of dithiothreitol, dithioerythritol, L-glutathione, and tris (2-carboxyethyl) phosphine hydrochloride. In some instances, the intercrosslinking agent comprises an oxidizing agent. In some instances, the oxidizing agent comprises at least one of horseradish peroxidase (HRP), sodium periodate, and silver nitrate. In some instances, the intercrosslinking agent induces self-crosslinking of functional groups present on at least one of the annealing component flowable microgel particles or annealing components to produce a crosslinkage. In some instances, the crosslinkage comprises at least one of a covalent bond, a coordination complex, a hydrogen bond, an electrostatic interaction, a cation-π interaction, a π-π stack, and a van der Waals interaction. In some instances, methods comprise contacting the first functionalized microgel particle and the second functionalized microgel particle with the intercrosslinking agent *in situ.* In some instances, methods comprise applying an external stimulus to the microporous scaffold to release the intercrosslinker. In some instances, applying an external stimulus to the microporous scaffold occurs indirectly by applying the external stimulus to tissue around the microporous scaffold. In some instances, the external stimulus is selected from light, an electromagnetic field, ultrasound, heat, cooling, and a combination thereof. In some instances, methods comprise incorporating a therapeutic agent into the stabilized scaffold. In some instances, incorporating comprises at least one of diffusing the therapeutic agent into the collection of flowable microgel particles; covalently linking the therapeutic agent to the flowable microgel particles; and photo-caging the therapeutic agent to the microgel particles. In some instances, incorporating comprises encapsulating the therapeutic agent in a nanoparticle, and mixing the therapeutic agent and the nanoparticle with the flowable microgel particles. In some instances, the nanoparticle and the therapeutic agent are lyophilized, comprising dissolving the nanoparticle and the therapeutic agent in aqueous buffer prior to mixing the nanoparticle and the therapeutic agent with the flowable microgel particles. In some instances, transferring and removing occur substantially simultaneously.

Disclosed herein, but not claimed , are methods of purifying flowable microgel particles comprising: obtaining a membrane filtration system; transferring flowable microgel particles from a first solvent to a second solvent, wherein the second solvent is immiscible with the first solvent, by controlled addition of a third solvent to the first solvent such that a single miscible phase containing the flowable microgel particles is maintained; and removing an impurity from the flowable microgel particles. In some instances, transferring and removing occur substantially simultaneously. In some instances, the membrane filtration system requires a single miscible phase for function. In some instances, the membrane filtration system is selected from tangential flow filtration (TFF), ultrafiltration-diafiltration (UFDF), microfiltration-diafiltration (MFDF), or hollow-fiber-diafiltration (HFDF). In some instances, the first solvent is a non-polar oil and the second solvent is water. In some instances, the third solvent is an alcohol solution. In some instances, the impurity is a surfactant.

Disclosed herein, but not claimed , are methods of concentrating flowable microgel particles in a solution or suspension comprising: pumping the flowable microgel particles through a membrane filtration system while a continuous phase volume is removed; continually concentrating the flowable microgel particles at a controlled membrane flux; and maintaining a wall shear stress inside the membrane filtration system. In some instances, the membrane filtration system is selected from tangential flow filtration (TFF), ultrafiltration-diafiltration (UFDF), microfiltration-diafiltration (MFDF), or hollow-fiber-diafiltration (HFDF). In some instances, the membrane flux is controlled between 100 and 1000 L/m²h. In some instances, the wall shear stress is maintained between 100s⁻¹ and 10,000s⁻¹. Any of the following figures that do not fall within the scope of the claims are provided for comparative purposes only.

### BRIEF DESCRIPTION OF THE DRAWINGS

Various aspects of the disclosure are set forth with particularity in the appended claims. A better understanding of the features and advantages of the present disclosure will be obtained by reference to the following detailed description that sets forth illustrative embodiments, in which the principles of the disclosure are utilized, and the accompanying drawings of which:
**FIG. 1** shows an exemplary application of a microporous gel disclosed herein to a wound void with a medical device implant. A syringe applicator of a solution of free-flowing microgel particles is applied to the wound void. Microgel particles are annealed using light energy to form a porous network. The porous network allows cells to migrate through the gel, with the result of improving the health of the wound-device interface.
**FIG. 2** shows an exemplary wound, wherein a microporous scaffold has been formed between the medical implant and the surrounding tissue. The presence of interconnected pores, with or without cells that have migrated into the microporous scaffold, are represented by the black color between the silver spherical shapes, the latter of which represent the microgel particles.
**FIG. 3** shows an exemplary method of controlling the release of diffusible molecules (active pharmaceutical ingredients) into the microporous gel. By combining multiple diffusion rates, dependent upon diffusion rates only (gel) and multiple mechanisms including enzymatic, hydrolytic, photonic, and thermal (nanoparticles), the microporous gel can achieve highly complex release profiles DIRECTLY to the cells growing through it (unlike any other scaffolding systems).
**FIG. 4** shows an exemplary schematic representation of pre-functionalization of flowable microgel particles.
FIG. 5 shows an exemplary schematic representation of post-functionalization of flowable microgel particles.
FIG 6 shows an exemplary schematic representation of *in situ* addition of a crosslinking agent.
FIG 7 shows an exemplary schematic representation of *in situ* addition of a crosslinking agent.
FIG. 8 shows an exemplary schematic diagram of flowable microgel particle synthesis by a water-in-oil emulsion and purification by tangential flow filtration.
FIG. 9 shows an exemplary workflow of purifying flowable microgel particles, aiming to maintain one miscible continuous phase with isopropanol, which is miscible with both oil and water, as an intermediate solvent to transfer the particles, initially dispersed in oil, into water, and finally to an aqueous buffer.
FIG. 10 shows characterization of wounds in pigs treated with a flowable microgel particle system disclosed herein five days after treatment. FIG. 10A shows multinucleated giant cell (MNGC) formation. FIG. 10B shows acute inflammation. FIG. IOC shows wound atrophy was reduced the microporous scaffold.
FIG. 11 shows characterization of wounds in pigs treated with a flowable microgel particle system disclosed herein fourteen days after treatment. FIG. 11A shows reepithelialization. FIG. 11B shows quantification of fibrosis.
FIG. 12 shows augmentation of wound healing vascularization with a flowable microgel particle system disclosed herein five days after treatment. FIG. 12A shows quantification of vessel ingrowth. FIG. 12B shows sizes of vessels formed. FIG. 12C shows the percentage of vessels larger than 10 µm.

### DETAILED DESCRIPTION OF THE DISCLOSURE

Medical devices, such as implants and surgical instruments, are used for a wide variety of applications. Use of these tools can be complicated by inflammation, infection, pain, scarring, and inability of an implant site or surgical site to heal or repair. The microporous gel systems disclosed herein may initially exist in a fluidic state, as a composition of flowable microgel particles in a solution. For example, in certain application, this solution is for application to the implant or surgical site in a subject before, after and/or concurrently with application of the medical device to improve the health and healing of the site. Due to its fluidic nature, the microporous gel system completely fills any space that may remain in the site surrounding the medical device. Once the solution and medical device are applied, the annealing agent is added or activated to anneal the microgel particles, creating a microporous scaffold. The microporous gel systems disclosed herein, unlike other porous gel systems, do not require porogens to produce the micropores of the scaffold. Instead, the microporous gel systems disclosed herein comprise microgel particles that are annealed and optionally crosslinked together while allowing for micropores to form between the microgel particles. Cells of the subject can, in certain applications, migrate through the micropores of the scaffold aiding in healing the site. By way of example, healing the site may comprise vascularizing, depositing extracellular matrix, and producing proteins and enzymes that aid in healing. In addition to aiding healing, the annealed scaffold may act as a functional glue to maintain the medical device placement in the site. The nature of the fluid-to-scaffold property *in vivo* provides a custom fit for the device; for example, a one-size-fits-all for the medical device. The microporous gel systems may also comprise therapeutic agents for treating the site for inflammation, pain or infection. The therapeutic agents include, anti-inflammatory agents, analgesics, and antimicrobials. Therapeutic agents specific to the site may also be used. For example, the medical implant may be a cardiac pacemaker, and a therapeutic agent specific to the implantation site may be an antimicrobial agent.

### Certain Terminologies

Unless defined otherwise, all technical and scientific terms used herein have the same meaning as is commonly understood by one of skill in the art to which the claimed subject matter belongs. It is to be understood that the foregoing general description and the following examples are exemplary and explanatory only and are not restrictive of any subject matter claimed. In this application, the use of the singular includes the plural unless specifically stated otherwise. It must be noted that, as used in the specification and the appended claims, the singular forms "a," "an" and "the" include plural referents unless the context clearly dictates otherwise. In this application, the use of "or" means "and/or" unless stated otherwise. Furthermore, use of the term "including" as well as other forms, such as "include", "includes," and "included," is not limiting.

As used herein, ranges and amounts can be expressed as "about" a particular value or range. About also includes the exact amount. For example, "about 5 µI_," means "about 5 µI_," and also "5 µL." Generally, the term "about" includes an amount that would be expected to be within experimental error. The term "about" includes values that are within 10% less to 10% greater of the value provided. For example, "about 50%" means "between 45% and 55%." Also, by way of example, "about 30" means "between 27 and 33."

The section headings used herein are for organizational purposes only and are not to be construed as limiting the subject matter described.

As used herein, the terms "individual(s)", "subject(s)" and "patient(s)" mean any mammal. In some embodiments, the mammal is a human. In some embodiments, the mammal is a non-human.

The term "statistically significant" or "significantly" refers to statistical significance and generally means a two standard deviation (2 SD) below normal, or lower, concentration of the marker. The term refers to statistical evidence that there is a difference. It is defined as the probability of making a decision to reject the null hypothesis when the null hypothesis is actually true. The decision is often made using the p-value. A p-value of less than 0.05 is considered statistically significant.

As used herein, the term "treating" and "treatment" refers to administering to a subject an effective amount of a composition so that the subject as a reduction in at least one symptom of the disease or an improvement in the disease, for example, beneficial or desired clinical results. For purposes of this invention, beneficial or desired clinical results include, alleviation of one or more symptoms, diminishment of extent of disease, stabilized *(e.g.,* not worsening) state of disease, delay or slowing of disease progression, amelioration or palliation of the disease state, and remission (whether partial or total), whether detectable or undetectable. Alternatively, treatment is "effective" if the progression of a disease is reduced or halted. Those in need of treatment include those already diagnosed with a disease or condition, as well as those likely to develop a disease or condition due to genetic susceptibility or other factors which contribute to the disease or condition, such as weight, diet and health of a subject are factors which may contribute to a subject likely to develop diabetes mellitus. Those in need of treatment also include subjects in need of medical or surgical attention, care, or management.

Without further elaboration, it is believed that one skilled in the art, using the preceding description, can utilize the present invention to the fullest extent. The following examples are illustrative only.

### Systems

Provided herein are systems comprising a microporous gel system as claimed . Microporous gel systems disclosed herein comprise a collection of flowable microgel particles and at least two annealing components. The microporous gel system may also simply be referred to herein as a "gel" or "hydrogel." Additionally, microporous gel systems disclosed herein may comprise a crosslinker that links the flowable microgel particles together via the annealing component. In general, resulting stabilized scaffolds comprise interstitial spaces therein. By way of example, medical devices include cardiac implantable electronic devices and neural implantable electronic devices.

Systems disclosed herein may comprise a container to contain the microporous gel system, *e.g.*, a bottle, tube, syringe, syringe barrel, or plastic bag. Systems disclosed herein may comprise an application device for applying the microporous gel system to a tissue defect. The container may be the application device, may be used with the application device, or may be used instead of the application device.

The collection of flowable microgel particles and annealing agents may be stored in a single container. The collection of flowable microgel particles and annealing agents may be administered from a single container. Additional components of the systems, such as crosslinkers, therapeutic agents, therapeutic agent releasing agents, nanoparticles, and cell adhesive peptides, including all those disclosed herein, may be stored or administered from the single container or a separate container.

The collection of flowable microgel particles may be stored in a first container and the annealing agents may be stored in a second container. The collection of flowable microgel particles may be administered from a first container and the annealing agents may be administered from a second container.

In examples not claimed , a first portion of the flowable microgel particles is administered from a first container and a second portion of the flowable microgel particles is administered from a second container. Contents of first and second containers may be administered sequentially. Contents of first and second containers may be administered simultaneously.

Any one of the systems disclosed herein may comprise an application device to apply the microporous gel system to a tissue of a subject. By way of example, the application device may comprise a syringe, a spatula, a squeezable tube, a cannula, or any combination thereof. The application device may comprise a needle. The needle may be blunt so as to avoid damaging or piercing a tissue. The microporous gel may have a viscosity low enough before annealing to be sprayed on the tissue of the subject. Thus, the application device may comprise a spray mechanism.

Containers and application devices disclosed herein encompass a wide range of volumes that are suitable for application to a wound, surgical or implant site receiving a medical device. Volumes include, about 0.1 mL to about 0.5 L, about 0.1 mL to about 0.2 L, about 0.1 mL to about 0.1 L, about 0.1 mL to about 75 mL, about 0.1 mL to about 60 mL, about 0.1 mL to about 50 mL, about 0.1 mL to about 25 mL, about 0.1 mL to about 20 mL, about 0.1 mL to about 10 mL, about 1 mL to about 0.5 L, about 1 mL to about 0.2 L, about 1 mL to about 1 L, about 1 mL to about 75 mL, about 1 mL to about 60 mL, about 1 mL to about 50 mL, about 1 mL to about 25 mL, about 1 mL to about 20 mL, or about 1 mL to about 10 mL.

### Microporous Gel Systems

Provided but not claimed are methods and systems for treating a condition in a subject in need thereof, comprising administering to the subject a microporous gel system disclosed herein. Microporous gel systems may also simply be referred to herein as a "gel" or "hydrogel." The microporous gel systems disclosed herein may take different forms, and, unless otherwise specified, the various terms that are used to reference these forms, such as microporous gel scaffold, stabilized scaffold, collection of flowable microgel particles, and microporous gel, may be used interchangeably herein. The microporous gel system may be administered to a site in the subject before, after or simultaneously with application of an implant or surgical device disclosed herein to the site. The microporous gel systems may be as claimed . Flowable microgel particles may also be referred to herein simply as "microgel particles." Methods of synthesizing flowable microgel particles are disclosed herein but not claimed.

### Flowable microgelparticles

The flowable microgel particles may be spherical particles or roughly spherical particles. The flowable microgel particles may have a diameter. The flowable microgel particles may have an average diameter of about 10 micrometers. The flowable microgel particles may have an average diameter of about 15 micrometers. The flowable microgel particles may have an average diameter of about 25 micrometers. The flowable microgel particles may have a diameter of about 50 micrometers. The flowable microgel particles may have an average diameter of about 100 micrometers. The flowable microgel particles may have an average diameter of about 150 micrometers. The flowable microgel particles may have an average diameter of about 200 micrometers. The flowable microgel particles may have a diameter within the range of about 10 micrometers to about 500 micrometers. The flowable microgel particles may have a diameter within the range of about 10 micrometers to about 200 micrometers. The flowable microgel particles may have a diameter within the range of about 15 micrometers to about 200 micrometers. The flowable microgel particles may have a diameter within the range of about 15 micrometers to about 150 micrometers. The flowable microgel particles may have a diameter within the range of about 30 micrometers to about 100 micrometers.

The diameter of the flowable microgel particles may depend on a component or property of a solvent in which they are dispersed before the microporous gel system becomes a stabilized scaffold. The solvent may be water. The solvent may be isotonic with blood of the subject. The solvent may be a saline solution. The solvent may be a buffered saline solution. In examples , the solvent is acidic. The solvent may have a pH of about 4 to about 7. The solvent may have a pH of about 3, about 4, about 5, about 6, or about 7. In examples , the solvent is alkaline. The solvent may have a pH greater than 7. The solvent may have a pH of about 8, about 9 or about 10.

### Backbonepolymers

Flowable microgel particles disclosed herein comprise at least one backbone polymer. By way of example, the backbone polymer may comprise a polymer selected from poly(ethylene glycol), hyaluronic acid, polyacrylamide, or polymethacrylate. The backbone polymer of the flowable microgel particles disclosed herein may comprise a hydrophilic polymer, amphiphilic polymer, synthetic or natural polymer ***{e.g*.,** poly(ethylene glycol) (PEG), poly(propylene glycol), poly(hydroxyethylmethacrylate), hyaluronic acid (HA), gelatin, fibrin, chitosan, heparin, heparan, and synthetic versions of HA, gelatin, fibrin, chitosan, heparin, or heparan). The backbone polymer of the flowable microgel particles disclosed herein may be made from any natural (e.g., modified HA) or synthetic polymer ***{e.g.,*** PEG) capable of forming a hydrogel. The backbone polymer may comprise a natural polymer containing nitrogen, such as proteins and derivatives, including crosslinked or modified gelatins, and keratins. The backbone polymer may comprise a vinyl polymer such as poly(ethyleneglycol) acrylate, poly(ethyleneglycol) methacrylate, poly(ethyleneglycol) vinyl sulfone, poly(ethyleneglycol) maleimide, poly(ethyleneglycol) norbornene, poly(ethyleneglycol) allyl. The backbone polymer may comprise a polyacrylamide or a polymethacrylates. The backbone polymer may comprise a polyester, a polyamide, a polyurethane, and a mixture or copolymer thereof. The backbone polymer may comprise a graft copolymer obtained by initializing polymerization of a synthetic polymer on a preexisting natural polymer.

The flowable microgel particles disclosed herein may, additionally to the backbone polymer, comprise a suitable support material. The support material may be suitable for most tissue engineering/regenerative medicine applications. The support material is generally biocompatible and preferably biodegradable. Examples of suitable support material include, natural polymeric carbohydrates and their synthetically modified, crosslinked, or substituted derivatives, such as gelatin, agar, agarose, crosslinked alginic acid, chitin, substituted and crosslinked guar gums, cellulose esters, especially with nitrous acids and carboxylic acids, mixed cellulose esters, and cellulose ethers; natural polymers containing nitrogen, such as proteins and derivatives, including crosslinked or modified gelatins, and keratins; vinyl polymers such as poly(ethyleneglycol)acrylate/methacrylate/vinyl sulfone/maleimide/norbornene/allyl, polyacrylamides, polymethacrylates, copolymers and terpolymers of the above polycondensates, such as polyesters, polyamides, and other polymers, such as polyurethanes; and mixtures or copolymers of the above classes, such as graft copolymers obtained by initializing polymerization of synthetic polymers on a preexisting natural polymer. A variety of biocompatible and biodegradable polymers are available for use in therapeutic applications; examples include: polycaprolactone, polyglycolide, polylactide, poly(lactic-co-glycolic acid) (PLGA), and poly-3- hydroxybutyrate.

The backbone polymer may be present at a concentration of about 1% w/v to about 15 % w/v of the microporous gel. The backbone polymer may be present at a concentration of 1% w/v to 15% w/v of the microporous gel. The backbone polymer may be present at a concentration of about 2% w/v to about 10% w/v of the microporous gel. The backbone polymer may be present at a concentration of 2% w/v to 10% w/v of the microporous gel. The backbone polymer may be present at a concentration of about 1% w/v of the microporous gel. The backbone polymer may be present at a concentration of about 2% w/v of the microporous gel. The backbone polymer may be present at a concentration of about 3% w/v of the microporous gel. The backbone polymer may be present at a concentration of about 4% w/v of the microporous gel. The backbone polymer may be present at a concentration of about 5% w/v of the microporous gel. The backbone polymer may be present at a concentration of about 6% w/v of the microporous gel. The backbone polymer may be present at a concentration of about 7% w/v of the microporous gel. The backbone polymer may be present at a concentration of about 8% w/v of the microporous gel. The backbone polymer may be present at a concentration of about 9% w/v of the microporous gel. The backbone polymer may be present at a concentration of about 10% w/v of the microporous gel. The backbone polymer may be present at a concentration of about 11% w/v of the microporous gel. The backbone polymer may be present at a concentration of 12% w/v of the microporous gel.

### Annealing Components

Microporous gel systems as claimed in claim 1 comprise a first and a second annealing component.

In many cases, annealing components are merely functional groups comprising a reactive moiety. By way of example, the reactive moiety may comprise at least one functional group selected from a vinyl sulfone, thiol, amine, imidazole, aldehyde, ketone, hydroxyl, azide, alkyne, vinyl, alkene, maleimide, carboxyl, *N*-hydroxysuccinimide (NHS) ester, isocyanate, isothiocyanate, hydroxylamine, and thione. The annealing component may comprise a vinyl group. The annealing component may comprise a free cysteine. The annealing component may comprise a thiol. The annealing component may comprise an amine. The annealing component may comprise a reactive moiety. The reactive moiety may comprise a catechol *(e.g.,* L-DOPA, dopamine). The reactive moiety may comprise sialic acid *(e.g.* neuraminic acid). The reactive moiety may comprise boronic acid *(e.g.,* 3-aminophenylboronic acid). The reactive moiety may comprise a molecular cage *(e.g.,* cyclodextrin, cucurbituril, calixarene, pillararene, crown ether, cavitand, cryptands carcerand). The reactive moiety may comprise adamantane. The reactive moiety may comprise biotin. The reactive moiety may comprise streptavidin.

Annealing components disclosed herein may include large biological molecules. The annealing component may comprise a peptide. The annealing component may consist essentially of a peptide. In some instances, the annealing component comprises a nucleic acid. The annealing component may consist essentially of a nucleic acid. The annealing component may comprise a protein. The annealing component may comprise an antibody or antigen binding antibody fragment. The annealing component may comprise an epitope. The annealing component may comprise an enzymatic substrate.

### Crosslinkers

Microporous gel systems disclosed herein may comprise at least one crosslinker. In some instances, at least a portion of the flowable microgel particles comprise a crosslinker. In some instances, at least a portion of the flowable microgel particles are interlinked by a crosslinker. The crosslinker may be an intracrosslinker, providing intracrosslinking (intracrosslinks) within the flowable microgel particles. The crosslinker may be an intercrosslinker, providing intercrosslinking (intercrosslinks) between flowable microgel particles. The crosslinker may be an extracrosslinker, providing extracrosslinking (extracrosslinks) between the flowable microgel particles and a substrate. The substrate may be tissue. The substrate may be a medical device.

Generally, crosslinkers disclosed herein comprise at least two functional groups. The crosslinker may comprise a first functional group and a second functional group. The first functional group and the second functional group may be the same. The first functional group and the second functional group may be different. Crosslinkers disclosed herein may also be referred to as multifunctionalized crosslinkers.

Crosslinkers may be degradable. Crosslinkers disclosed herein may comprise a peptide. Crosslinkers disclosed herein may comprise an amino acid. Crosslinkers may comprise a nonpeptide polymer. Degradable crosslinkers may also be random sequences, Omi target sequences, Heat-Shock Protein target sequences. The crosslinker may comprise an amino acid having D chirality. The crosslinker may comprise an amino acid having L chirality. Crosslinkers may comprise hydrolytically degradable natural and synthetic polymers consisting of the same backbones listed above ***{e.g.*,** heparin, alginate, poly(ethyleneglycol), polyacrylamides, polymethacrylates, copolymers and terpolymers of the listed polycondensates, such as polyesters, polyamides, and other polymers, such as polyurethanes). The crosslinker may be synthetically manufactured or naturally isolated. The crosslinker may comprise DNA oligonucleotides with sequences corresponding to: restriction enzyme recognition sequences, CpG motifs, Zinc finger motifs, CRISPR or Cas-9 sequences, Talon recognition sequences, and transcription factor-binding domains. The crosslinker may be activated on at least two ends by a reactive group, defined as a chemical group allowing the crosslinker to participate in the crosslinking reaction to form a polymer network or gel (intracrosslinking within particles) or to anneal particles together (intercrosslinking between particles) or to anneal the particles to a substrate (extracrosslinking between particles and a substrate), where these functionalities can include: cysteine amino acids, synthetic and naturally occurring thiol-containing molecules, carbene-containing groups, vinyl-containing groups, activated esters, acrylates, norborenes, primary amines, hydrazides, phosphenes, azides, epoxy-containing groups, SANPAH containing groups, and diazirine containing groups. In some instances, flowable microgel particles themselves may act as crosslinkers.

### Intracrosslinkers

In some instances, intracrosslinkers disclosed herein are crosslinkers that participate in the crosslinking reaction to form microgel particles. Often, the intracrosslinker is functionalized with two or more functional groups. By way of example, the functional groups of the intracrosslinker may be selected from a vinyl sulfone, a thiol, an amine, an imidazole, an aldehyde, a ketone, a hydroxyl, an azide, an alkyne, a vinyl, an alkene, a maleimide, a carboxyl, a N-Hydroxysuccinimide (NHS) ester, an isocyanate, an isothiocyanate, ahydroxylamine, and a thione. The intracrosslinker may be homofunctional (same functional groups) or heterofunctional (different functional groups). Examples of crosslinking reactions carried out by intracrosslinker include,

Michael addition, amide bond coupling, "click" chemistry (e.g. Diels-Alder cycloaddition, Huisgen 1,3 -dipolar cycloaddition), reductive amination, carbamate linkage, ester linkage, thioether linkage, disulfide bond, hydrazone bond, oxime coupling, thiourea coupling. By way of example, an intracrosslinker may be a matrix metalloprotease (MMP)-degradable crosslinker. Examples of MMP-degradable crosslinkers are synthetically manufactured or naturally isolated peptides with sequences corresponding to MMP-1 target substrate, MMP-2 target substrate, MMP-9 target substrates. An intracrosslinker may be a dithiol-poly(ethylene glycol). An intracrosslinker may be a diamine-poly(ethylene glycol). An intracrosslinker may be a diamine-poly(ethylene glycol). An intracrosslinker may be a 4-ARM-poly(ethylene glycol)-thiol. An intracrosslinker may be a 4-ARM-poly(ethylene glycol)-vinyl sulfone. An intracrosslinker may be a 8-ARM-poly(ethylene glycol)-thiol. An intracrosslinker may be a 8-ARM-poly(ethylene glycol)-vinyl sulfone.

### Intercrosslinkers

In some instances, intercrosslinkers disclosed herein that participate in the crosslinking reaction between particles to anneal particles together. Often, the intercrosslinker is functionalized with two or more functional groups. By way of example, the functional groups of the intercrosslinker may be selected from a vinyl sulfone, a thiol, an amine, an imidazole, an aldehyde, a ketone, a hydroxyl, an azide, an alkyne, a vinyl, an alkene, a maleimide, a carboxyl, a N-Hydroxysuccinimide (NHS) ester, an isocyanate, an isothiocyanate, ahydroxylamine, and a thione. The multifunctionalized crosslinker may be homofunctional (combination of same functional groups) or heterofunctional (combination of different functional groups). Examples of crosslinking reactions carried out by intercrosslinker include,

Michael addition, amide bond coupling, "click" chemistry (e.g. Diels-Alder cycloaddition, Huisgen 1,3 -dipolar cycloaddition), reductive amination, carbamate linkage, ester linkage, thioether linkage, disulfide bond, hydrazone bond, oxime coupling, thiourea coupling. An intercrosslinker may be a dithiol-poly(ethylene glycol). An intercrosslinker may be a diamine-poly(ethylene glycol). An intercrosslinker may be a dithiololigo(ethylene glycol). An intercrosslinker may be a diamine-oligo(ethylene glycol). An intercrosslinker may be an ethylenediamine. An intercrosslinker may be a butylenediamine.

### Extracrosslinkers

In some instances, extracrosslinkers disclosed herein participate in the crosslinking reaction between particles and a substrate (particle-substrate annealing). By way of example, the functional groups of the extracrosslinker may be selected from a vinyl sulfone, a thiol, an amine, an imidazole, an aldehyde, a ketone, a hydroxyl, an azide, an alkyne, a vinyl, an alkene, a maleimide, a carboxyl, a N-Hydroxysuccinimide (NHS) ester, an isocyanate, an isothiocyanate, ahydroxylamine, and a thione. The extracrosslinker may be homofunctional (same functional groups) or heterofunctional (different functional groups). Examples of crosslinking reactions carried out by extracrosslinkers disclosed herein include Michael addition, amide bond coupling, "click" chemistry (*e.g.* Diels-Alder cycloaddition, Huisgen 1,3-dipolar cycloaddition), reductive amination, carbamate linkage, ester linkage, thioether linkage, disulfide bond, hydrazone bond, oxime coupling, thiourea coupling. By way of example, an extracrosslinker may be a matrix metalloprotease (MMP)-degradable crosslinker. Examples of MMP-degradable crosslinkers are synthetically manufactured or naturally isolated peptides with sequences corresponding to MMP-1 target substrate, MMP-2 target substrate, MMP-9 target substrates. An extracrosslinker may be a dithiol-poly(ethylene glycol). An extracrosslinker may be a diamine-poly(ethylene glycol). An extracrosslinker may be a diamine-poly(ethylene glycol). An extracrosslinker may be a 4-ARM-poly(ethylene glycol)-thiol. An extracrosslinker may be a 4-ARM-poly(ethylene glycol)-vinyl sulfone. An intracrosslinker may be a 8-ARM-poly(ethylene glycol)-thiol. An extracrosslinker may be a 8-ARM-poly(ethylene glycol)-vinyl sulfone.

### Annealing Agents

Provided herein are microporous gel systems comprising at least two annealing agents disclosed herein. The annealing agent may be a crosslinking agent disclosed herein. The annealing agent may comprise a photoinitiator. By way of example, the photoinitiator may be Eosin Y. The annealing agent may be triethanolamine. The annealing agent may be a transglutaminase enzyme. The annealing agent may be enzyme Factor XIII. The annealing agent may comprise a free radical transfer agent. The annealing agent may comprise an electron transfer agent. Examples of additional and alternative annealing agents, by way of example, include active esters and nucleophiles, catechols that crosslink upon oxidation, and other redox sensitive molecules.

### Crosslinking Agents

Microporous gel systems may comprise a crosslinking agent. The crosslinking agent may be an intracrosslinking agent for providing intracrosslinks within flowable microgel particles. In general, intracrosslinking agents do not form crosslinks ***(e.g.*,** they are not part of the bonds), but instead initiate intracrosslinking reactions between intracrosslinkers. The crosslinking agent may be an intercrosslinking agent for providing intercrosslinks between flowable microgel particles. The crosslinking agent may be an extracrosslinking agent for providing extracrosslinks between flowable microgel particles and a substrate. A crosslinking agent may comprise a reducing agent. Examples of reducing agents are dithiothreitol, dithioerythritol, L-glutathione, and tris (2-carboxyethyl) phosphine hydrochloride. Crosslinking agents disclosed herein may comprise an oxidizing agent. Examples of oxidizing agents are horseradish peroxidase (HRP), sodium periodate, and silver nitrate. Crosslinking agents disclosed herein may comprise a metal complexing agent. Crosslinking agents disclosed herein may comprise a catalyst. The crosslinking agent may be a base. Examples of bases are triethylamine, triethanolamine, 4-dimethylaminopyridine, triphenylphosphine. The crosslinking agent may induce self-crosslinking of the annealing components present on the flowable microgel particles. Resulting crosslinkages, by way of example, may comprise at least one of a covalent bond, a coordination complex, a hydrogen bond, an electrostatic interaction, a cation-π interaction, a π-π stacking, and a van der Waals interaction.

### Cell Adhesive Peptides

Microporous gel systems may comprise a cell adhesive peptide disclosed herein. The flowable microgel particles may comprise a cell adhesive peptide. The cell adhesive peptide may be any peptide that promotes adherence of a cell to the microgel particles. The cell adhesive peptide may be at least a portion of an extracellular matrix protein. The cell adhesive peptide may be at least a portion of a collagen. The cell adhesive peptide may be at least a portion of a fibronectin. The cell adhesive peptide may be an integrin. The cell adhesive peptide may be a ligand to a receptor expressed on the cell. The cell adhesive peptide may be a cluster of differentiation (CD) protein. The cell adhesive peptide may be a naturally-occurring peptide. The cell adhesive peptide may be a synthetic peptide. The cell adhesive peptide may be homologous to the naturally-occurring peptide. The cell adhesive peptide may be at least about 70% homologous to a naturally-occurring peptide. The cell adhesive peptide may be at least about 80% homologous to a naturally-occurring peptide. The cell adhesive peptide may be at least about 90% homologous to a naturally-occurring peptide. The cell adhesive peptide may be at least 70% homologous to a naturally-occurring peptide. The cell adhesive peptide may be at least 80% homologous to a naturally-occurring peptide. The cell adhesive peptide may be at least 90% homologous to a naturally-occurring peptide. The cell adhesive peptide may be on a surface of the microgel particle. By way of example, the cell adhesive peptide may comprise tripeptide Arginine-Glycine-Aspartate (RGD). The cell adhesive peptide may comprise K peptide (K peptide: Ac-FKGGERCG-NH2). The cell adhesive peptide may comprise Q peptide (Q peptide: Ac-NQEQVSPLGGERCG-NH2).

### Microporous Scaffolds

As one of skill in the art would understand from the instant disclosure, microporous gel systems, or components thereof, as disclosed herein, may be initially fluidic in nature and eventually become a non-fluidic, microporous scaffold that provide a buffer between a medical device and a tissue. The non-fluidic, microporous scaffold may be referred to herein simply as a "microporous scaffold." The microporous scaffold may be flexible or compressible, with a foam or sponge-like quality. The microporous scaffold may be more rigid than a foam or sponge, in order to provide more support to an implanted medical device. The gel before annealing may have a compressive modulus (mechanical stiffness) of about 200-1000 Pa. The gel before annealing may have a compressive modulus (mechanical stiffness) of about 200-500 Pa. The gel before annealing may have a compressive modulus (mechanical stiffness) of about 500-1000 Pa. Once annealed, the gel may have a compressive modulus of about 1,500 Pa to about 200,000 Pa. Once annealed, the gel may have a compressive modulus of about 1,500 Pa to about 10,000 Pa. Once annealed, the gel may have a compressive modulus of about 10,000 Pa to about 50,000 Pa. Once annealed, the gel may have a compressive modulus of about 50,000 Pa to about 125,000 Pa. Once annealed, the gel may have a compressive modulus of about 125,000 Pa to about 200,000 Pa.

The microporous scaffold may be non-fluidic due to reactions that take place during or after the application of the microporous gel system components. The reactions may result in production of a covalent bond between two or more annealing components disclosed herein. Such a microporous scaffold may be referred to herein as a "stabilized scaffold." By way of example, reactions that may result in a covalent bond include Michael addition, amide bond coupling, "click" chemistry reactions *(e.g.* Diels-Alder cycloaddition, Huisgen 1,3-dipolar cycloaddition), reductive amination, carbamate linkage, ester linkage, thioether linkage, oxime coupling, and thiourea coupling. Alternatively or additionally, reactions may result in production of a non-covalent bond between two or more flowable microgel particles. By way of example, reactions that may result in a non-covalent bond include electrostatic interactions, hydrogen bonding, cation-π, π-π stacking, metal-ligand binding, and van der Waals interactions.

Microporous scaffolds disclosed comprise at least one of a bond, a linkage, an interaction, a coupling and a connection between flowable microgel particles. The bond, linkage, interaction, coupling or connection is between two annealing components.

In some instances, the bond is a covalent bond. In some instances, the bond is a non-covalent bond. In some instances, the bond is selected from an amide bond, an imine bond, an ester bond, a C-C bond through Michael addition, a disulfide bond, a hydrazone bond, a hydrogen bond, and a metal ligand bond. In some instances, the ester bond comprises a cyclic boronate ester. In some instances, the linkage is selected from a carbamate linkage, an ester linkage, and a thioether linkage. In some instances, the coupling is selected from an oxime coupling, and a thiourea coupling. In some instances, the interaction is selected from an electrostatic interaction and a van der Waals interaction. In some instances, the bond, linkage, interaction, coupling or connection is a result of a reaction between two functional groups. Nonlimiting examples of such functional groups include a vinyl sulfone, a thiol, an amine, an imidazole, an aldehyde, a ketone, a hydroxyl, an azide, an alkyne, a vinyl, an alkene, a maleimide, a carboxyl, a N-Hydroxysuccinimide (NHS) ester, an isocyanate, an isothiocyanate, a hydroxylamine, a thione.

The interstitial spaces within the stabilized scaffold of microgel particles may occupy about 10% of the total volume of the stabilized scaffold. The interstitial spaces within the stabilized scaffold of microgel particles may occupy about 20% of the total volume of the stabilized scaffold. The interstitial spaces within the stabilized scaffold of microgel particles may occupy about 30% of the total volume of the stabilized scaffold. The interstitial spaces within the stabilized scaffold of microgel particles may occupy about 40% of the total volume of the stabilized scaffold. The interstitial spaces within the stabilized scaffold of microgel particles may occupy about 10% to about 40% of the total volume of the stabilized scaffold. within the stabilized scaffold of microgel particles may occupy about 15% to about 40% of the total volume of the stabilized scaffold. within the stabilized scaffold of microgel particles may occupy about 20% to about 40% of the total volume of the stabilized scaffold.

### Microporous Gel System Stability

The microporous gel systems disclosed herein may have a shelf life of at least about six months. The microporous gel systems disclosed herein may have a shelf life of at least about seven months. The microporous gel systems disclosed herein may have a shelf life of at least about eight months. The microporous gel systems disclosed herein may have a shelf life of at least about nine months. The microporous gel systems disclosed herein may have a shelf life of at least about ten months. The microporous gel systems disclosed herein may have a shelf life of at least about eleven months. The microporous gel systems disclosed herein may have a shelf life of at least about one year. The microporous gel systems disclosed herein may have a shelf life of at least about fourteen months. The microporous gel systems disclosed herein may have a shelf life of at least about sixteen months. The microporous gel systems disclosed herein may have a shelf life of at least about eighteen months. The microporous gel systems disclosed herein may have a shelf life of at least about twenty months. The microporous gel systems disclosed herein may have a shelf life of at least about twenty-two months. The microporous gel systems disclosed herein may have a shelf life of at least about two years. The microporous gel systems disclosed herein may have a shelf life of at least about three years. The microporous gel systems disclosed herein may have a shelf life of at least about four years. The microporous gel systems disclosed herein may have a shelf life of at least about five years. The microporous gel systems disclosed herein may have a shelf life of at least six months. The microporous gel systems disclosed herein may have a shelf life of at least seven months. The microporous gel systems disclosed herein may have a shelf life of at least eight months. The microporous gel systems disclosed herein may have a shelf life of at least nine months. The microporous gel systems disclosed herein may have a shelf life of at least ten months. The microporous gel systems disclosed herein may have a shelf life of at least eleven months. The microporous gel systems disclosed herein may have a shelf life of at least one year. The microporous gel systems disclosed herein may have a shelf life of at least fourteen months. The microporous gel systems disclosed herein may have a shelf life of at least sixteen months. The microporous gel systems disclosed herein may have a shelf life of at least eighteen months. The microporous gel systems disclosed herein may have a shelf life of at least twenty months. The microporous gel systems disclosed herein may have a shelf life of at least twenty-two months. The microporous gel systems disclosed herein may have a shelf life of at least two years. The microporous gel systems disclosed herein may have a shelf life of at least three years. The microporous gel systems disclosed herein may have a shelf life of at least four years. The microporous gel systems disclosed herein may have a shelf life of at least five years.

The microporous gel systems disclosed herein, or components thereof, may be stable (e.g., have a shelf life) up to a temperature disclosed herein. The microporous gel systems disclosed herein, or components thereof, may be stable at up to a temperature when in a dissolved state, a fluidic state, a lyophilized state, or a dehydrated state. The microporous gel systems, or components thereof, may be stable at room temperature (about 25°C). The microporous gel systems, or components thereof, may be stable at 25°C. The microporous gel systems, or components thereof, may be stable at about 25°C to about 35°C. The microporous gel systems, or components thereof, may be stable at 25°C to 35°C. The microporous gel systems, or components thereof, may be stable up to about 35°C, up to about 40°C, up to about 45°C, up to about 50°C, up to about 55°C, up to about 60°C, up to about 65°C, up to about 70°C, up to about 75°C, up to about 80°C, up to about 85°C, up to about 90°C, up to about 95°C, up to about 100°C, up to about 105°C, up to about 110°C, up to about 115°C, up to about 120°C, up to about 125°C, up to about 130°C, up to about 135°C, up to about 140°C, up to about 145°C, or up to about 150°C. The microporous gel systems, or components thereof, may be stable up to 35°C, up to 40°C, up to 45°C, up to 50°C, up to 55°C, up to 60°C, up to 65°C, up to 70°C, up to 75°C, up to 80°C, up to 85°C, up to 90°C, up to 95°C, up to 100°C, up to 105°C, up to 110°C, up to 115°C, up to 120°C, up to 125°C, up to 130°C, up to 135°C, up to 140°C, up to 145°C, or up to 150°C.

In some instances the stability or shelf life of the microporous gel system is increased by storing the microporous gel system, or a component thereof, below room temperature. Below room temperature may be about 20°C to about -80°C, about 20°C to about -20°C, about 20°C to about 0°C, or about 20°C to about 4°C. Below room temperature may be 20°C to -80°C, 20°C to -20°C, 20°C to 0°C, or 20°C to 4°C.

The microporous gel systems disclosed herein may have a shelf life of at least about one year at about 25°C. The microporous gel systems disclosed herein may have a shelf life of at least about one year at about 4°C. The microporous gel systems disclosed herein may have a shelf life of at least about one year at about 25°C to about 35°C. The microporous gel systems disclosed herein may have a shelf life of at least about one year at about 4°C to about 35°C. The microporous gel systems disclosed herein may have a shelf life of at least one year at 25°C. The microporous gel systems disclosed herein may have a shelf life of at least one year at 4°C. The microporous gel systems disclosed herein may have a shelf life of at least one year at 25°C to 35°C. The microporous gel systems disclosed herein may have a shelf life of at least one year at 4°C to 35°C.

### Medical Devices

Provided but not claimed are methods and systems for treating a condition in a subject in need thereof, comprising administering to the subject a medical device disclosed herein. The medical device may be for administration to a site in the subject before, after or simultaneously with application of a microporous gel system disclosed herein. The medical device may at least partially contain the microporous gel system. The medical device may be coated with the microporous gel system. Medical devices of many different shapes and sizes will be compatible with the microporous gel systems and stabilized scaffolds disclosed herein. Due to the initial fluidic nature of the microporous gel systems disclosed herein, the microporous gel system can coat portions or shapes of various medical devices before it becomes a stabilized scaffold. In some aspects, the stabilized scaffold is conformed to the shape and size of the device. In some aspects, the stabilized scaffold is adapted to the shape and size of the device. For the same reason that the microporous gel system is compatible with medical devices of many shapes and sizes *(e.g.,* its fluidic nature), it is also compatible with implant sites of various shapes and sizes. Thus, the microporous gel system can adapt to, conform to, or custom fill various implant sites before it becomes the stabilized scaffold.

The medical device may be an implant. The implant may be a temporary implant. A temporary implant may be an implant that remains in the subject for more than one day, but not more than one week. A temporary implant may be an implant that remains in the subject for more than one week, but not more than one month. The implant may be a permanent implant. The implant may be an organ, artificial or donor. The implant may be a biomaterial, such as a mesh or fabric. The implant may be a printed device or tissue. As used herein, an implant is a medical device that is administered to a subject that remains in the subject after administration. The implant may be functional due to its physical structure. The implant may be functional due to an active function that it performs. The implant may comprise a glucose sensor. The implant may comprise a glucose dispenser. The implant may comprise a cell-based therapy delivered in a device (e.g., an islet cell transplantation).

The medical device may be a surgical device. As used herein, a surgical device is a structure that is used in the subject during a procedure, and that does not remain in the subject after the procedure. By way of example, the surgical device may be a laser, scalpel or needle. The procedure may be a surgical procedure. The surgical procedure may comprise a modification of a tissue of the subject. The modification may comprise cutting the tissue. The procedure may be a non-surgical procedure. By way of example, the non-surgical procedure may comprise insertion of a catheter or application of an ostomy device.

The medical device may be a vascular stent. The medical device may be a prosthetic device. The medical device may be an orthopedic implant, such as an artificial knee, meniscus, hip, elbow or portion thereof. The medical device may be a dental implant. The medical device may be a breast implant. The medical device may be a spinal implant, such as a screw, rod or artificial disc. The medical device may be an intra-uterine device. The medical device may be an ear tube. The medical device may be an artificial eye lens.

Provided herein are systems for treating a heart arrhythmia in a subject in need thereof . The Cardiac Implantable Electronic Device (CIED) may be a device that is capable of correcting or improving an abnormal heart rhythm. CIEDs may include, cardiac pacemakers and implantable cardioverter defibrillators.

Provided but not claimed are methods and systems for treating a condition in a subject in need thereof, comprising administering to the subject a Neural Implantable Electronic Device (NIED), and a microporous gel system disclosed herein. NIEDs include, a neural implant, a brain implant, and a spinal implant. The implant may also be referred to as neural stimulator or prosthetic. The microporous scaffolds disclosed herein may provide an interface between the NIED and a neuron or a brain. The microporous scaffolds disclosed herein may provide an interface between the NIED and subcutaneous or connective tissue. The NIED may comprise an electrode. NIEDs may include computer chips, an electro echocardiogram array, a spinal cord stimulator. The NIED may be a device that produces a deep brain stimulation. The NIED may be a device that produces a vagus nerve stimulation. The NIED may be a neuroimaging device or a neurological activity recording device. The NIED may be a brainstem implant. The NIED may be a device that is placed in or on a brain. The NIED may be placed in a sensory organ *(e.g.*, ear, eye, nose, brain, skin). The NIED may be placed in a spine or brain stem of a subject. The NIED may be a device that is placed in or on an eye. The NIED may be a device that is placed in or on an ear, such as a cochlear implant, by way of example. The NIED may comprise a computer chip. The microporous scaffolds disclosed herein may provide an interface between the computer chip and a neuron or a brain (brain-computer interface). The NIED may be a device that stimulates, blocks or records signals from neurons. The NIED may be a device that re-wires the brain or re-wires neurons in the subject. Re-wiring may comprise forming or blocking a neural synapse.

The medical devices disclosed herein may be connected to a computer or in communication with a computer. The medical devices disclosed herein may be battery operated. The medical devices disclosed herein may be connected or in communication with a recording device, a stimulating device, an electrical device, a power source, a computer, a controller, or any combination thereof.

Medical devices disclosed herein may comprise a coating. In some instances, medical devices disclosed herein do not comprise a coating. In some instances, medical devices disclosed herein are pre-coated with a coating. In some instances, the coating comprises a coating functional group that acts as an annealing component. In some instances, the coating comprises a coating functional group that is capable of binding a flowable microgel particle disclosed herein. In some instances, some instances, the coating comprises a coating functional group that is capable of reacting with a flowable microgel particle disclosed herein. In some instances, the coating comprises a coating functional group that is capable of binding an annealing component disclosed herein. In some instances, the coating comprises a coating functional group that is capable of reacting with an annealing component disclosed herein. In some instances, the coating comprises a coating functional group that is capable of binding a crosslinker disclosed herein. In some instances, the coating comprises a coating functional group that is capable of reacting with a crosslinker disclosed herein. In some instances, the coating functional group is an annealing component. The functional group may become a part of an extracrosslink between the medical device and the flowable microgel particle.

Systems disclosed herein may comprise a device coating agent, wherein the device coating agent enables coating of the microporous gel system to the medical device. The systems disclosed herein may comprise a device coating agent, wherein the device coating agent promotes coating of the microporous gel system to the medical device. The systems disclosed herein may comprise a device coating agent, wherein the device coating agent enables adhesion of the microporous gel system to the medical device. The systems disclosed herein may comprise a device coating agent, wherein the device coating agent promotes adhesion of the microporous gel system scaffold to the medical device.

Systems disclosed herein may comprise a device coating agent, wherein the device coating agent enables adhesion of the stabilized scaffold to the medical device. The systems disclosed herein may comprise a device coating agent, wherein the device coating agent promotes adhesion of the stabilized scaffold to the medical device. The device coating agent may be applied to the medical device. The device coating agent may be a component of the microporous gel system. The device coating agent may be mixed with the microporous gel system or component thereof, prior to use. The device coating agent may comprise a ceramic, also referred to in the art as a bioceramic or a bioglass. The device coating agent may comprise a polymer. The polymer may comprise polyethylene glycol. The polymer may comprise a polyvinyl group. The polymer may comprise a parylene. The polymer may comprise a poly-N-vinylpyrrolidone ) ( PNP). The polymer may comprise a polyurethane. The polymer may comprise hyaluronan or hyaluronic acid.

### Therapeutic Agents

Provided herein are systems comprising a therapeutic agent disclosed herein.

Examples of therapeutic agents are anti-inflammatory agents, antimicrobial agents, and analgesics. The therapeutic agent may be incorporated in the flowable microgel particles. The therapeutic agent may be incorporated in the flowable microgel particles before forming the stabilized scaffold. The therapeutic agent may be mixed with the flowable microgel particles and/or annealing agent before forming the stabilized scaffold. The therapeutic agent may be incorporated in the stabilized scaffold after forming the stabilized scaffold. The therapeutic agent may be released from the stabilized scaffold into or on to the site or tissue of the subject. For example, the therapeutic agent may be incorporated in the stabilized scaffold and released as the stabilized scaffold is degraded in the tissue or as the stabilized scaffold is infiltrated by cells of the tissue or subject. The therapeutic agent may be released either by an internal trigger such as tissue mediated and/or enzyme mediated hydrolysis, hydrolysis not mediated by tissue or enzymes, enzymolysis, redox change, temperature change or by an external trigger such as light, electromagnetic field, ultrasound. Alternatively or additionally, the system may comprise a therapeutic agent. The therapeutic agent may be released from the stabilized scaffold by addition of therapeutic agent-releasing agent. The therapeutic agent may be connected to or contained within a nanoparticle or nanoparticle system disclosed herein. In some instances, the medical device comprises a therapeutic agent disclosed herein. Systems disclosed herein may comprise a single therapeutic agent or a combination of a plurality of therapeutic agents.

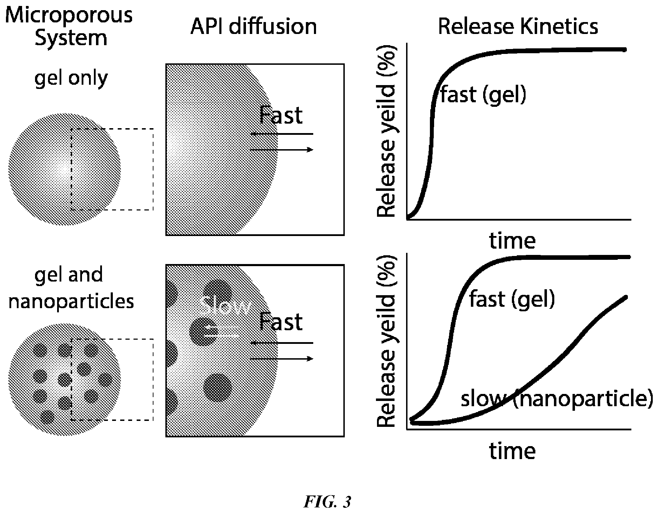

Provided herein are systems comprising a therapeutic agent, wherein the therapeutic agent is incorporated in the stabilized scaffold and released from the stabilized scaffold. In certain embodiments, the therapeutic agent is released from the stabilized scaffold at more than one rate, see, *e.g.,* **FIG. 3****.** In certain embodiments, the therapeutic agent is actively released. In some embodiments, the therapeutic agent is passively released, also referred to as "diffused." The therapeutic agent may be released in less than about one day. The therapeutic agent may be released in less than about a week. The therapeutic agent may be released in less than about one month. The therapeutic agent may be released in less than one day. The therapeutic agent may be released in less than a week. The therapeutic agent may be released in less than one month. At least a portion of the therapeutic agent may be released in less than about one day. A least a portion of the therapeutic agent may be released in less than about one week. At least a portion of the therapeutic agent may be released in less than about one month. At least a portion of the therapeutic agent may be released in less than one day. At least a portion of the therapeutic agent may be released in less than one week. At least a portion of the therapeutic agent may be released in less than one month. The therapeutic agent may be released from the stabilized scaffold over a period of about 1 day to about 1 week. The therapeutic agent may be released from the stabilized scaffold over a period of about 1 day to about 2 weeks. The therapeutic agent may be released from the stabilized scaffold over a period of about 1 day to about 3 weeks. The therapeutic agent may be released from the stabilized scaffold over a period of about 1 day to about 100 days. At least a portion of the therapeutic agent may be released over a period of about 1 day to about 100 days. The therapeutic agent may be released from the stabilized scaffold over a period of 1 day to 1 week. The therapeutic agent may be released from the stabilized scaffold over a period of 1 day to 2 weeks. The therapeutic agent may be released from the stabilized scaffold over a period of 1 day to 3 weeks. The therapeutic agent may be released from the stabilized scaffold over a period of 1 day to 100 days. At least a portion of the therapeutic agent may be released over a period of 1 day to 100 days. The portion of the therapeutic agent may be about 1% to about 50% of the therapeutic agent. The portion of the therapeutic agent may be about 10% to about 50% of the therapeutic agent. The portion of the therapeutic agent may be about 10% to about 80% of the therapeutic agent. The portion of the therapeutic agent may be about 1% to about 10%. The portion of the therapeutic agent may be 1%) to 50%) of the therapeutic agent. The portion of the therapeutic agent may be 10% to 50% of the therapeutic agent. The portion of the therapeutic agent may be 10% to 80% of the therapeutic agent. The portion of the therapeutic agent may be 1% to 10%.

The therapeutic agent may be present in the microporous gel system at a concentration of about 1 ≈g/mL to about 1 mg/mL. The therapeutic agent may be present in the microporous gel system at a concentration of about 1 µg/mL, about 5 µg/mL, about 10 µg/mL, about 20 µg/mL, about 30 µg/mL, about 40 µg/mL, about 50 µg/mL, about 60 µg/mL, about 70 µg/mL, about 80 µg/mL, about 90 µg/mL, or about 100 µg/mL. The therapeutic agent may be present in the microporous gel system at a concentration of about 100 µg/mL, about 200 µg/mL, about 300 µg/mL, about 400 µg/mL, about 500 µg/mL, about 600 µg/mL, about 700 µg/mL, about 800 µg/mL, about 900 µg/mL, or about 1 mg/mL. The therapeutic agent may be present in the microporous gel system at a concentration of about 1 mg/mL to about 10 mg/mL. The therapeutic agent may be present in the microporous gel system at a concentration of about 10 mg/mL to about 350 mg/mL. The therapeutic agent may be present in the microporous gel system at a concentration of about 50 mg/mL to about 300 mg/mL. The therapeutic agent may be present in the microporous gel system at a concentration of about 5 mg/ mL, about 10 mg/ mL, about 20 mg/mL, about 40 mg/mL, about 60 mg/mL, about 80 mg/mL, about 100 mg/mL, about 150 mg/ mL, about 200 mg/mL, about 250 mg/mL, about 300 mg/mL, about 350 mg/mL, or about 400 mg/mL. The therapeutic agent may be present in the microporous gel system at a concentration of 1 ≈g/mL to 1 mg/mL. The therapeutic agent may be present in the microporous gel system at a concentration of 1 µg/mL, 5 µg/mL, 10 µg/mL, 20 µg/mL, 30 µg/mL, 40 µg/mL, 50 µg/mL, 60 µg/mL, 70 µg/mL, 80 µg/mL, 90 µg/mL, or 100 µg/mL. The therapeutic agent may be present in the microporous gel system at a concentration of 100 µg/mL, 200 µg/mL, 300 µg/mL, 400 µg/mL, 500 µg/mL, 600 µg/mL, 700 µg/mL, 800 µg/mL, 900 µg/mL, or 1 mg/mL. The therapeutic agent may be present in the microporous gel system at a concentration of 1 mg/mL to 10 mg/mL. The therapeutic agent may be present in the microporous gel system at a concentration of 10 mg/mL to 350 mg/mL. The therapeutic agent may be present in the microporous gel system at a concentration of 50 mg/mL to 300 mg/mL. The therapeutic agent may be present in the microporous gel system at a concentration of 5 mg/ mL, 10 mg/ mL, 20 mg/mL, 40 mg/mL, 60 mg/mL, 80 mg/mL, 100 mg/mL, 150 mg/ mL, 200 mg/mL, 250 mg/mL, 300 mg/mL, 350 mg/mL, or 400 mg/mL.

Provided herein are systems that comprise anti-inflammatory agents, and combinations thereof . The anti-inflammatory agent may be a steroidal or hormonal agent. The anti-inflammatory agent may be a non-steroidal anti-inflammatory agent. The anti-inflammatory agent may be synthetic or non-naturally-occurring. The anti-inflammatory agent may be naturally-occurring. By way of example, the anti-inflammatory agent may be ibuprofen, aspirin, natural or synthetic corticosteriods, an anti-inflammatory neuropeptide a-melanocyte stimulating hormone (a-MSH), dexamethasone, or meloxicam, or combinations thereof.

Provided herein are systems that comprise antimicrobial agents, combinations thereof, and uses thereof. The antimicrobial agent may be an antibacterial agent, also referred to as an antibiotic. The antimicrobial agent may be selected from an antibacterial agent, an antifungal agent, an antimycotic agent, an antiparasitic agent, or an antiseptic agent. By way of example, the antibiotic may be a *β*-lactam *(e.g.,* penam, cephem, monobactam, carbapenem, penicillin, ceftriaxone), a macrolide *(e.g.,* erythromycin), an aminoglycoside *(e.g.,* tobramycin, neomycin, ampicillin, aminopenicillin, amoxicillin, kanamycin), a glycopeptide antibiotic *(e.g.,* vancomycin), a quinolone *(e.g.,* ciprofloxacin levofloxacin, moxifloxacin), a tetracycline, a phenicol or a sulfonamide.

Provided herein are systems comprising a combination of antimicrobial agents. The combination of antimicrobial agents may comprise a combination of antibiotics. The combination of antibiotics may be selected from a combination of a β-lactam, a macrolide, an aminoglycoside, a glycopeptide antibiotic, a quinolone, a tetracycline, a phenicol, a sulfonamide. The antibiotic may be present in the microporous gel system or the stabilized scaffold at a minimal inhibitory concentration. The antibiotic may be present in the microporous gel system or the stabilized scaffold at a concentration that is bactericidal at the implant site or surgical site. The antibiotic may be present in the microporous gel system or the stabilized scaffold at a concentration that is bacteriostatic at the implant site or surgical site.

Provided herein are systems comprising an antimicrobial agent, wherein the antimicrobial agent is incorporated in the stabilized scaffold and released from the stabilized scaffold. The antimicrobial agent may be released from the stabilized scaffold at a rate. In certain embodiments, the antimicrobial agent is released from the stabilized scaffold at more than one rate, see, *e.g.,* **FIG. 3****.** In certain embodiments, the antimicrobial agent is actively released. In some embodiments, the antimicrobial agent is passively released, also referred to as "diffused." The antimicrobial agent may be an antibiotic. The antimicrobial agent may be released over the period of at least about one week. The antimicrobial agent may be released over the period of at least about ten days. The antimicrobial agent may be released over the period of at least about two weeks. The antimicrobial agent may be released over the period of at least about three weeks. The antimicrobial agent may be released over the period of at least about four weeks. The antimicrobial agent may be released over the period of one week. The antimicrobial agent may be released over the period of ten days. The antimicrobial agent may be released over the period of two weeks. The antimicrobial agent may be released over the period of three weeks. The antimicrobial agent may be released over the period of four weeks. The antimicrobial agent may be released at a rate that reduces or kills microbes at the implant site or surgical site. The amount of antimicrobial agent, and the rate at which it is released at the implant site or surgical site, may be considered bactericidal. The antimicrobial agent may be released at a rate that maintains microbe presence at the implant site or surgical site, but prevents growth of microbes at the implant site or surgical site. The amount of antimicrobial agent, and the rate at which it is released at the implant site or surgical site, may be considered bacteriostatic.

Provided herein are systems that comprise an agent that prevents, alleviates or reduces pain or discomfort at the implant site or surgical site. Provided herein are systems that comprise at least one analgesic, combinations of analgesics, or a use thereof. By way of example, the analgesic may be paracetamol (also known as acetaminophen), an opioid, a non-steroidal anti-inflammatory drug (NSAID), a cyclooxygenase inhibitor, a cannabinoid, a ketamine, and a combination thereof. Alternatively, or additionally, the systems may comprise a local anesthetic or a use thereof. By way of example, the local anesthetic may be benzocaine, chloroprocaine, cyclomethycaine, dimethocaine/larocaine, piperocaine, propoxycaine, procaine/novocaine, proparacaine, tetracaine/amethocaine, chloroprocaine, saxitoxin, neosaxitoxin, tetrodotoxin, menthol, or eugenol, or a combination thereof. In certain embodiments, the system comprises a combination of the local anesthetic with a vasoconstrictor, or a used thereof. An example of a vasoconstrictor is epinephrine. Provided but not claimed are therapeutic agents disclosed herein and methods of incorporating the therapeutic agents into the microporous gel systems disclosed herein. The therapeutic agent may be directly incorporated in the microporous gel system disclosed herein. For example, the therapeutic agent may be loaded into or on to the microgel particles of the microporous gel system. The therapeutic agent may be passively loaded into the microporous gel via diffusion. The therapeutic agent may be passively loaded into the microporous gel via entrapment. The therapeutic agent may be directly incorporated in the microporous gel system by a covalent linkage between the therapeutic agent and a polymer or crosslinker of the microporous gel system. The therapeutic agent may be directly incorporated in the microporous gel system by immobilization of the therapeutic agent via a photo-caging method. The therapeutic agent may be loaded in a nanoparticle (a therapeutic agent-loaded nanoparticle). The microporous gel systems disclosed herein may comprise a mixture of therapeutic agent-loaded microparticles and therapeutic agent-loaded nanoparticles embedded into the microgel particles. Methods for incorporating therapeutic agent-loaded nanoparticles into the microporous gel system may comprise dissolving lyophilized therapeutic agent-loaded nanoparticles in an aqueous buffer prior to mixing the therapeutic agent-loaded nanoparticles with the microporous gel system. Methods for incorporating therapeutic agent-loaded nanoparticles into the microporous gel system may comprise directly embedding the therapeutic agent-loaded nanoparticles into the microgel particles during the microgel fabrication. The microporous gel systems disclosed herein may comprise a mixture of therapeutic agent-loaded microparticles and therapeutic agent-loaded nanoparticles embedded into the microgel particles.

Provided herein are systems that comprise a microporous gel system disclosed herein, wherein the microporous gel system releases a therapeutic agent disclosed herein into a tissue or biological fluid of a subject. Release of therapeutic agents may occur via passive diffusion from the microgel particles and/or nanoparticles (see, *e.g.,* **FIG. 3****).** Release of therapeutic agents may occur via an active release. The active release may be initiated by an external stimulus. The external stimulus, by way of example, may be light *(e.g.,* UV or NIR), a change of temperature, ultrasound, or a magnetic field. The active release may be initiated by an internal stimulus. The internal stimulus may be produced by the subject. The internal stimulus, by way of example, may be a pH change, a redox reaction, enzymatic activity, or chemical activity.

Therapeutic agents disclosed herein may be delivered in nanoparticles. By way of example, nanoparticles may comprise a polymer selected from poly(lactic-co-glycolic acid) (PLGA) or a copolymer thereof, a poly(anhydride), a poly(amide), a poly(ortho ester), a polycaprolactone. The nanoparticle may comprise hyaluronic acid. The nanoparticle may comprise chitosan. The nanoparticle may be a mesoporous silica nanoparticle. The nanoparticle may comprise a polymer with a lower critical solution temperature (LCST), such as poly(N-isopropylacrylamide) (PNIPAm) or co-polymer of PNIPAm, by way of example. The nanoparticle may comprise a polymer with an upper critical solution temperature (UCST) such as poly(hydroxyethylmethacrylate) (PHEMA) or polyethylene oxide (PEO) or poly(ethyleneoxide)-poly(propyleneoxide)-poly(ethyleneoxide) (PEO-PPO-PEO). The nanoparticle may comprise a self-immolating polymer such as poly(p-aminobenzyl oxycarbonyl) (poly(PABC)) that is capped with a cage that can be released upon a stimulus *(e.g.,* light, temperature, pH, redox, enzyme). In self-immolating systems, a single cleavage event of an end-cap can trigger an entire chain to degrade into small molecules, allowing the entrapped drug to be released. For instance, silver nitrate (used as an oxidizing agent) can be encapsulated into thermosensitive liposomes (made of 90 mol% dipalmitoyl phosphatidylcholine and 10 mol% of 1-palmitoyl lysophosphatidyl choline). The silver nitrate-loaded liposomes are entrapped in DOPA-functionalized microgel particles and are stable at room temperature. By way of example, nanoparticles may comprise liposomes or lipid vesicles. At body temperature, the lipid bilayer of the liposomes is more permeable allowing silver nitrate to be released and oxidize the catechol of the DOPA moieties into reactive quinones that can further react with another DOPA group leading to the crosslinking of the DOPA-functionalized microgel particles.

Provided herein are systems that comprise a core-shell nanoparticle system in which the shell responds to a stimulus. The core or shell may comprise any combination of the materials that are components of the nanoparticles disclosed herein. This core shell system may enable the nanoparticles to retain a cargo, such as a therapeutic agent disclosed herein, while being stored in an aqueous environment *(e.g.,* inside the microgel solution in a syringe). The cargo may be released by applying an external stimulus to the tissue site *(e.g.,* light, electromagnetic radiation). The cargo may be released by an internal stimulus, present in a tissue of the subject, *(e.g.,* enzymes, redox potential, pH, temperature). The microporous gel system may comprise a first portion of microgel particles and a second portion of microgel particles, wherein the first portion of microgel particles comprises the core-shell nanoparticle system, and wherein the second portion of microgel particles comprises an agent that dissolves the shell and initiates release of the cargo from the first portion of microgel particles. The methods disclosed but not claimed may comprise delivering the first portion and the second portion of microgel particles simultaneously or sequentially, through a single or multiple syringes or multi-barrel syringe/cannula/tube systems.

### Methods and Systems for Producing Microporous Gel Systems

Provided but not claimed are methods and systems for producing microporous gel systems. One of skill in the art understands that methods for producing microporous gel systems described herein may be performed with manufacturing systems comprising reagents and materials employed by the methods. In examples not claimed , the methods comprise synthesizing flowable microgel particles. The term "flowable microgel particle," as described herein, includes a hydrogel particle. Generally, flowable microgel particles disclosed herein comprise a high water content and "intra-crosslinks" (crosslinks within the particles). In general, a high water content is a water content greater than 50% to up to about 99.9% water. In examples not claimed , the water content is about 60% to about 99.9%. In examples not claimed , the water content is about 70% to about 99.9%. The intra-crosslinks may be physical, chemical, or a combination thereof.

### Synthesis of Flowable Microgel Particles

The microgel particles may be synthesized using a microfluidic device (one particle at a time per channel). The microgel particles may be synthesized by water-in-oil emulsion as described in greater detail herein. The microgel particles may be synthesized by water-in-oil emulsion with mechanical stirring. The microgel particles may be synthesized by water-in-oil emulsion using a static mixer. The microgel particles may be synthesized using in-line flowthrough synthesis. The microgel particles may be synthesized using a parallel production method (multiple particles at a time per channel). Methods of synthesizing flowable microgel particles disclosed herein are described in further detail throughout the instant disclosure.

Methods disclosed but not claimed may comprise synthesizing microgel particles by a water-in-oil emulsion process. In examples not claimed , the methods being with obtaining an oil or an oil mixture. By way of example, the oil may be a light mineral oil (LMO) or a heavy mineral oil (HMO). In examples not claimed , oil mixtures comprise a surfactant. Different surfactants can be employed. The surfactant may be a nonionic surfactant. Examples of nonionic surfactants are Span80, Span20, Tween20, Tween40, Tween60, Tween80, and tocopheryl polyethylene glycol 1000 succinate (TPGS). The surfactant may be an anionic surfactant. Examples of anionic surfactants are sodium dodecyl sulfate (SDS), sodium lauryl ether sulfate (SLES), and perfluorooctanesulfonate. The surfactant may be a cationic surfactant. Examples of cationic surfactants are cetyltrimethylammonium bromide (CTAB), and hexadecylpyridium bromide. The surfactant may be an amphoteric surfactant. Examples of amphoteric surfactants are betaine citrate, lauryl betaine, sodium, and (carboxymethyl) dimethyloleyl ammonium hydroxide. The concentration of the surfactant may vary from 0.01 to 5% v/v.

In examples not claimed , methods comprise adding the surfactant to the oil. In examples not claimed , methods comprise adding the surfactant to the oil prior to the addition of an aqueous solution/mixture to the oil. In examples not claimed , methods comprise adding the surfactant to an aqueous solution/mixture described herein. In examples not claimed , having a surfactant in the aqueous phase is beneficial because if the surfactant has a high-water solubility it is easy to remove during purification.

The oil or oil mixture may be added to a bioreactor vessel through a micron filter and stirred. In examples not claimed , the bioreactor vessel contains a volume from about 100 milliliters to about 1 liter. In examples not claimed , the bioreactor vessel contains a volume from about 1 liter to about 10 liters. In examples not claimed , the bioreactor vessel contains a volume from about 10 liters to about 100 liters. In examples not claimed , the bioreactor vessel contains a volume from about 100 liters to about 1000 liters. In examples not claimed , the bioreactor vessel contains a volume from about 100 liters to about 10,000 liters. In examples not claimed , the bioreactor vessel contains a volume from about 10 liters to about 1000 liters. In examples not claimed , the bioreactor vessel contains a volume from about 1000 liters to about 10,000 liters. In examples not claimed, the micron filter has a pore size of about 0.1 µm to about 1 µm. In examples not claimed , the micron filter has a pore size of about 0.2 µm.

In examples not claimed , methods of synthesizing microgel particles comprise modifying a backbone polymer. In examples not claimed , methods comprise attaching one or more functional groups to the backbone polymer. In examples not claimed , only one functional group is attached, and the resulting backbone polymer is referred to as a polymerization monomer. In some instances, two or more functional groups are attached, and the resulting backbone polymer is referred to as an intracrosslinking component. The term, "intracrosslinking component," as used herein, generally refers to molecules that participate in the formation of the intracrosslinks (they form the crosslink bonds). In examples not claimed , an intracrosslinking component is also an intracrosslinker.

In examples not claimed , methods of synthesizing microgel particles comprise mixing two or more types of intracrosslinking components. In examples not claimed , methods of synthesizing microgel particles comprise mixing two or more types of intracrosslinking components and an intracrosslinking agent. In examples not claimed , methods of synthesizing microgel particles comprise mixing an intracrosslinking component and an intracrosslinking agent. In examples not claimed, methods of synthesizing microgel particles comprise mixing a polymerizing agent and a polymerization monomer.

In examples not claimed , methods comprise obtaining a solution of at least one of an intracrosslinking component. In examples not claimed , methods comprise preparing a solution of at least one of an intracrosslinking component. In examples not claimed , methods comprise including an intracrosslinking agent in the solution. In examples not claimed , an intracrosslinking agent is not required because the intracrosslining component(s) self-crosslink without a crosslinking agent. Methods may comprise filtering the solution. The solution may comprise a backbone polymer. The solution may comprise a peptide. The solution may comprise a buffer or buffering agent. The solution may comprise a base catalyst.

By way of example, a solution comprising backbone polymer: 4-arm poly(ethylene glycol) functionalized with four vinyl sulfone groups (PEG-VS) and limiting amounts of K-peptide (Ac-FKGGERCG-NH2), Q-peptide (Ac-NQEQVSPLGGERCG-NH2), and RGD (Ac-RGDSPGERCG-NH2); may be mixed with MMP-degradable peptide with thiol-containing cysteines on the N and C termini. Both the functionalized PEG-VS and MMP-degradable peptide provide intracrosslinking components; PEG-VS provides four vinyl sulfone groups and MMP-degradable peptide provides two thiol groups. Upon mixing of the PEG-VS and MMP-degradable peptide (both intracrosslinking components) in the presence of triethanolamine, a base catalyst and intracrosslinking agent, intracrosslinking takes place and particles are formed. K-peptide (Ac-FKGGERCG-NH2), Q-peptide (Ac-NQEQVSPLGGERCG-NH2), and RGD (Ac-RGD SPGERCG-NH2); intracrosslinker:; and base catalyst: triethanolamine.

The concentration of 4-arm poly(ethylene glycol) vinyl sulfone (PEG-VS) (20 kDa) may be about 5%w/v to about 15%w/v. The concentration of 4-arm poly(ethylene glycol) vinyl sulfone (PEG-VS) may be about 10%w/v. The PEG-VS may be PEG-VS (20 kDa). The concentration of K-peptide may be about 100 µM to about ImM. The concentration of K-peptide may be about 500 µM. The concentration of Q-peptide may be about 100 µM to about ImM. The concentration of Q-peptide may be about 500 µM. The concentration of RGD may be about 0.1 mM to about 10 mM. The concentration of RGD may be about 1 mM. The concentration of triethanolamine may be about 50 mM to about 500 mM. The concentration of triethanolamine may be about 300 mM. The pH of triethanolamine may be about 7 to about 9. The pH of triethanolamine may be about 7.75.

In examples not claimed , methods comprise obtaining an intracrosslinker solution. In examples not claimed , methods comprise preparing an intracrosslinker solution. The intracrosslinker solution may comprise a degradable peptide. The intracrosslinker solution may comprise di-cysteine MMP-sensitive peptide. The concentration of the intracrosslinker in the intracrosslinker solution may be about 1 mM to about 50 mM. The concentration of the intracrosslinker in the intracrosslinker solution may be about 5 mM to about 15 mM. The method may comprise filtering the intracrosslinker solution.

Methods of producing may comprise mixing the intracrosslinking agent with the crosslinker solution to produce an aqueous mixture. In examples not claimed , methods comprise adding the aqueous mixture to the oil in an oil container. In examples not claimed , methods comprise adding an aqueous mixture to oil at a volume fraction of the aqueous phase into the oil phase. The volume fraction may be about 1% to about 10% v/v/ w/o. In examples not claimed , methods comprise injecting the aqueous mixture into the oil. Injecting may comprise the use of a peristaltic pump. In examples not claimed , the peristatic pump is operated at about 100 mL/min to about 200 mL/min. In examples not claimed, the peristatic pump is operated at about 150 mL/min. In some instances, the peristatic pump is operated at about 135 mL/min. In examples not claimed, injecting is performed immediately after mixing. The methods may comprise stirring the oil as the aqueous mixture is added. The speed of stirring (agitation) may vary from 100-20,000 rpm, depending upon the size of the reaction vessel and the size of particles needed. Different impeller types may be used for the agitation (turbine overhead stirrer, paddle overhead stirrer, blade stirrer, dissolver stirrer, spiral stirrer, propeller stirrer, double impeller). Stirring may occur for at least 1 hour. Stirring may occur for at least 2 hours. Stirring may occur for about 1 hour to about 24 hours. When stirring stops, flowable microgel particles settle to the bottom of the oil container. The final size of the flowable microgel particles may be dependent upon the concentrations of the materials, the speed of stirring, volume fraction, and speed of injection of the aqueous phase to the oil phase.

In examples not claimed, the methods may comprise a base-catalyzed Michael addition. The base may be an amine (e.g. triethanolamine, trimethylamine), an amidine (e.g. 1,8-diazabicycloundec-7-ene (DBU), 1,5-diazabicyclo[4.3.0]non-5-ene (DBN)), an imidazole, a pyridine (e.g. 4-dimethylaminopyridine (DMAP)), an amine (e.g., «-pentylamine), a phosphine (e.g. tri-n-propylphosphine, dimethylphenylphosphine, methyldiphenylphosphine, triphenylphosphine). In examples not claimed , the base is added to the oil prior to addition of an aqueous phase to oil. In examples not claimed, the base is added to a solution disclosed herein prior to formation of an aqueous mixture. In examples not claimed, it is beneficial to add the base to the oil because it ensures initiation of the gelation occurs only when the aqueous mixture is added to the oil.

In examples not claimed , methods comprise synthesizing flowable microgel particles by pumping the aqueous mixture into the oil and mixing the resulting emulsion with a static mixer connected to a reaction vessel. The flowable microgel particles that are generated by the static mixer are collected in the reaction vessel and kept under gentle agitation until the reaction is complete. In this method, a base catalyst may be added to the oil to prevent a gelation reaction from occurring in the aqueous mixture before particle dispersion in the static mixer. Benefits of using a static mixer include the following: gelation may occur when both phases are in contact in the static mixer; less oil is required; and a greater particle concentration is obtained. Thus, large manufacturing scales are achievable.

### Purifying Flowable Microgel Particles

Methods disclosed but not claimed, may comprise purifying flowable microgel particles. In examples not claimed , methods comprise synthesizing and purifying flowable microgel particles simultaneously. In examples not claimed, methods comprise purifying flowable microgel particles after synthesizing the flowable microgel particles. In examples not claimed, purifying flowable microgel particles comprises performing membrane separation of the flowable microgel particles from unwanted components. Different types of filtration membranes may be used (e.g., hollow fiber membranes with different pore sizes, different lumen IDs or flat sheet membrane). In examples not claimed , membrane separation comprises tangential flow filtration (TFF). In examples not claimed , membrane separation comprises ultrafiltration-diafiltration (UFDF). In examples not claimed , membrane separation comprises microfiltration-diafiltration (MFDF). ). In examples not claimed , membrane separation comprises hollow-fiber-diafiltration (UFDF). TFF generally comprises a membrane filtration and separation technique. TFF may be used herein to purify and concentrate flowable microgel particles. TFF may comprise generating a feed stream of a solution of flowable microgel particles that passes parallel to a membrane face. One portion of the solution may pass through the membrane (permeate) while the remainder (retentate) is recirculated back to the feed reservoir. This system may be referred to as diafiltration. This system may allow molecules (in the permeate) smaller than the membrane pores to move toward and through the membrane while the larger molecules, such as the flowable microgel particles, remain in the retentate. In examples not claimed, the flow in the filtration system may be controlled by a peristaltic pump. In examples not claimed , the flow in the filtration system may be controlled by a Quattroflow pump or any positive displacement pump. In examples not claimed, the filtration system may be closed to surrounding environment. In examples not claimed , the filtration system may be open to surrounding environment.

Methods of purifying may comprise removing excess oil from the flowable microgel particles. Methods of purifying may comprise dispersing the particles in an alcohol solution. Methods of purifying may comprise removing excess oil and surfactant that are not miscible in water while keeping the particles (mainly composed of water) dispersed and sufficiently swollen and ensuring no particle aggregation. Methods of purifying may comprise slowly transferring the particles into an aqueous buffer while preventing the surfactant from precipitating. Transferring rate may be linked to the flux of filtrate passing through the membrane, and occur at a rate of about 1 to about 1000 LMH (liters/m²h). Transferring may occur at a rate of about 100 to about 500 LMH. Transferring may occur at a rate of about 200 to about 300 LMH. This transition rate may be particularly important to ensure that a surfactant does not precipitate on to (and within) the flowable microgel particles, rendering the particles unsuitable for a microporous scaffold. The transition rate may achieve at least one of (i) particle hydrogel mesh swelling, which is a product of the affinity for certain solvents for a given hydrogel polymer backbone/crosslinker system, and (ii) solubility of the surfactant in the continuous phase outside of the particle.

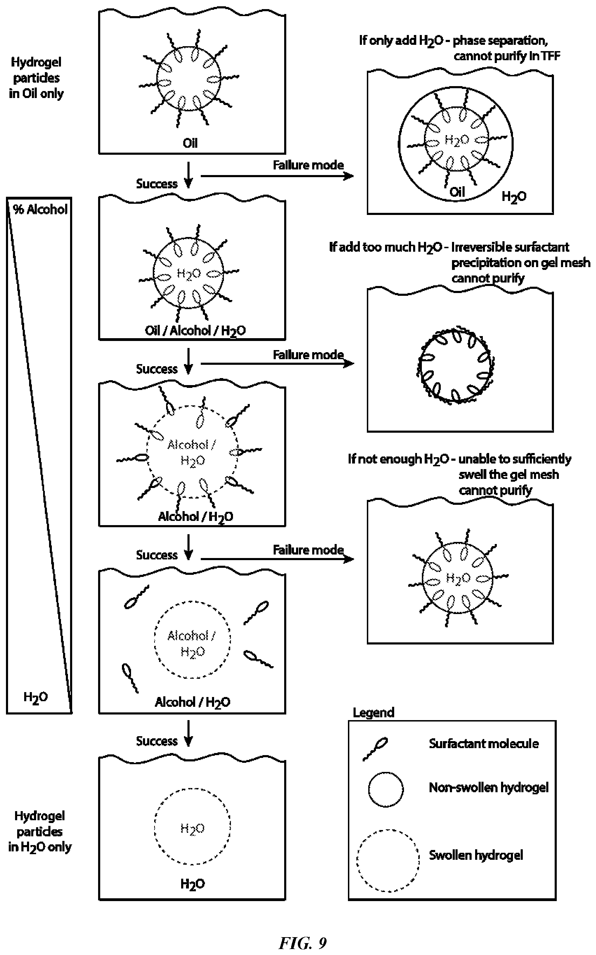

Methods may comprise ensuring that there is only one miscible continuous phase to allow TFF to proceed by using an intermediate solvent (e.g. isopropanol (IPA)) which is miscible with both mineral oil and water, and capable of substantially swelling the particle mesh when mixed with either the mineral oil or the water. This can enable the transfer of the oildispersed particles from the oil into water, while removing surfactant and finally to an aqueous buffer, while never creating more than one miscible continuous phase. See, *e.g.,* **FIG. 9****.**

In examples not claimed , methods comprise performing membrane filtration or membrane separation to concentrate the flowable microgel particles to a particle concentration in a solution, fluid, or solvent described herein. The shear rate occurring on the inside face of the membrane filter as solution passes by may affect the capability of concentrated particle suspensions to flow, and become increasingly difficult to maintain flow at high concentrations. In examples not claimed , the shear rate may be between Is⁻¹ and 100s⁻¹. In examples not claimed , the shear rate may be between 100s⁻¹ and 500s⁻¹. In examples not claimed , the shear rate may be between 500s⁻¹ and 1,000s⁻¹. In examples not claimed , the shear rate may be between 1,000s⁻¹ and 5,000s⁻¹. In examples not claimed , the shear rate may be between 5,000s⁻¹ and 10,000s⁻¹. The concentration may be about 1% v/v to about 100% v/v. The concentration may be about 1%> v/v to about 10%> v/v. The concentration may be about 10%) v/v to about 20% v/v. The concentration may be about 20% v/v to about 30%> v/v. The concentration may be about 30%> v/v to about 40% v/v. The concentration may be about 40% v/v to about 50%) v/v. The concentration may be about 50% v/v to about 60% v/v. The concentration may be about 60% v/v to about 70% v/v. The concentration may be about 70% v/v to about 80% v/v. The concentration may be about 80% v/v to about 90% v/v. The concentration may be about 90% v/v to about 99% v/v. The concentration may be about 90% v/v to about 100% v/v. Additionally or alternatively, flowable microgel particles are concentrated by centrifugation.

In examples not claimed , methods comprise contacting the flowable microgel particles with at least one solvent to purify the particles. In examples not claimed, methods comprise contacting the flowable microgel particles with a gradient of solvents. In examples not claimed, the solvent is selected from an alcohol solution, water, and an aqueous buffer. In examples not claimed, the solvent is an organic solvent (including alcohol solutions). Organic solvents may be suitable for transitioning flowable microgel particles from an oil phase to an aqueous phase. Organic solvents include isopropyl alcohol (IPA), methanol, ethanol, glycerol, acetone, acetonitrile, hexane, tetrahydrofuran, and 1,4-dioxane. In examples not claimed , a combination of various organic solvents can be used. By way of example, a combination of organic solvents may comprise hexane and IPA. In examples not claimed, the alcohol solution comprises IPA, also referred to as isopropanol. In examples not claimed , the alcohol solution consists of **IPA** and water. In examples not claimed , the alcohol solution is about 30%, about 35%, about 40%, about 45%, about 50%, about 55%, about 60%, about 65%, about 70%, about 75%, about 80%, about 85%, or about 90% alcohol. In examples not claimed , the alcohol solution is about 90%, about 92%, about 95%, about 98%), or about 99% alcohol. In examples not claimed, methods comprise performing diafiltration with a 95%) IPA solution. In examples not claimed, methods comprise performing diafiltration with a 50%) **IPA** solution. In examples not claimed , methods comprise performing diafiltration with *100%* pure water. Examples of aqueous buffers include phosphate buffers, HEPES buffers, MES buffers, Tris buffers, Tricine buffers, PIPES buffers, borate buffers, MOPS buffers, and combinations thereof. In examples not claimed , the aqueous buffer has a pH of about 6.6, about 6.0, about 6.2, about 6.4, about 6.6, about 6.8, about 7.0, about 7.2, about 7.4, about 7.6, about 7.8, about 8.0, about 8.2, about 8.4, about 8.6, about 8.8, or about 9.0. By way of example, the aqueous buffer may comprise 10 mM phosphate buffer, 100 mM NaCl, and 5 µM Eosin Y, with a pH of 7.4.

In examples not claimed , methods do not comprise initially adding only water to oil in which the flowable microgel particles are present. Adding water directly to oil would likely create two immiscible phases, in which the particles stick at an interface (along with precipitated surfactant), and foul separation membranes. In examples not claimed, methods comprise initially adding an organic solvent and gradually transitioning to water through a gradient of solutions comprising decreasing amounts of organic solvent in water. In this way, a single miscible phase is maintained.

In examples not claimed , methods of producing microgel particles comprise producing a mesh of polymers, peptides, or a combination thereof within the microgel particles. The mesh may be described as a three dimensional network formed by intracrosslinks within the microgel particle. The mesh is porous, resulting in pores within the particles. Pores within the microgel particles are generally nanoscopic pores, as opposed to the microscopic pores between the particles. Nanoscopic may be considered to be less than one micron in its greatest dimension. Microgel particles may swell in size when they are transitioning from organic solvent into water. Since the microparticles grow in size when they swell, polymer chains that make up the microgel particle may unfold in the water, thereby increasing the spacing within the 3D network (the mesh) and increasing the space between each neighboring polymer in the mesh (the pore size).

### Annealing Flowable Microgel Particles

In examples not claimed, the methods comprise annealing two or more flowable microgel particles together. This may be referred to as particle - particle annealing. Particle-particle annealing includes intercrosslinking. In examples not claimed, annealing results in formation of at least one bond. In some instances, the bond is a covalent bond. Examples of a covalent bond are bonds found in an amide, ester, C-C bond through Michael addition, carbamate, disulfide bond, oxime, thiourea, hydrazone, and imine. In examples not claimed, the bond is a non-covalent bond. Examples of non-covalent bonds are those found in an interaction such as, electrostatic interactions, hydrogen bonding, cation-π, π-π stacking, metal-ligand binding, and van der Waals interactions. In examples not claimed , the methods comprise linking two or more flowable microgel particles together. Examples of linking reactions include Michael addition, amide bond coupling, "click" chemistry *(e.g.* Diels-Alder cycloaddition, Huisgen 1,3-dipolar cycloaddition), reductive amination, carbamate linkage, ester linkage, thioether linkage, disulfide bonding, hydrazone bonding, oxime coupling, and thiourea coupling.

In examples not claimed, methods disclosed herein comprise mixing flowable microgel particles that contain at least two annealing component. In general, annealing components comprise a reactive moiety. Examples of annealing components include catechol *(e.g.,* L-DOPA, dopamine), sialic acid *(e.g.,* neuraminic acid), boronic acid *(e.g.*, 3-aminophenylboronic acid), a molecular cage *(e.g.,* cyclodextrins, cucurbiturils, calixarenes, pillararenes, crown ethers, cavitands, cryptands, carcerands), adamantane, biotin, and streptavidin. Additional examples of annealing components are described herein. In examples not claimed , at least one annealing component is part of a backbone polymer of a microgel particle. In examples not claimed, at least one annealing component is initially separate from the backbone polymer. In examples not claimed , at least one annealing component is part of the intracrosslinker of a microgel particle. In examples not claimed , methods comprise adding at least one annealing component to the flowable microgel particle, which may be referred to herein as "functionalizing" the flowable microgel particle or producing a "functionalized microgel particle." In examples not claimed, mixing may occur *in vitro.* In examples not claimed , mixing may occur *ex vivo.* In examples not claimed , mixing may occur *in vitro* immediately before being applied to a subject or medical device. In examples not claimed , mixing may occur *ex vivo* immediately before being applied to a subject or medical device. In examples not claimed , mixing may occur *in situ.* In examples not claimed , methods comprise applying multiple flowable microgel particles, annealing components, and additional system components described herein *(e.g.,* therapeutic agents, annealing agents, etc.) with a multi-compartment applicator *(e.g.,* multi-barrel syringe) to keep these components separate until application or immediately there before.

Methods disclosed herein may comprise annealing a first flowable microgel particle to a second flowable microgel particle, wherein the first flowable microgel particle has a first functional group (first annealing component) and the second flowable microgel particle has a second functional group (second annealing component), wherein the first functional group and the second functional group are different. Methods may comprise mixing the first flowable microgel particle and the second flowable microgel particle such that the first functional group reacts with the second functional group to form a bond. This may be referred to as "hetero-annealing." One advantage to hetero-annealing is that an external annealing agent or crosslinker is not required.

In examples not claimed , the annealing component is part of the intracrosslinking component. In examples not claimed , the annealing component is part of the intracrosslinker of a microgel particle. In examples not claimed , the second annealing component is part of the backbone polymer of a microgel particle. In examples not claimed, the intracrosslinking component is part of the annealing component. In examples not claimed , the intracrosslinker of a microgel particle is part of the annealing component. In examples not claimed , the backbone polymer of a microgel particle is part of the second annealing component.

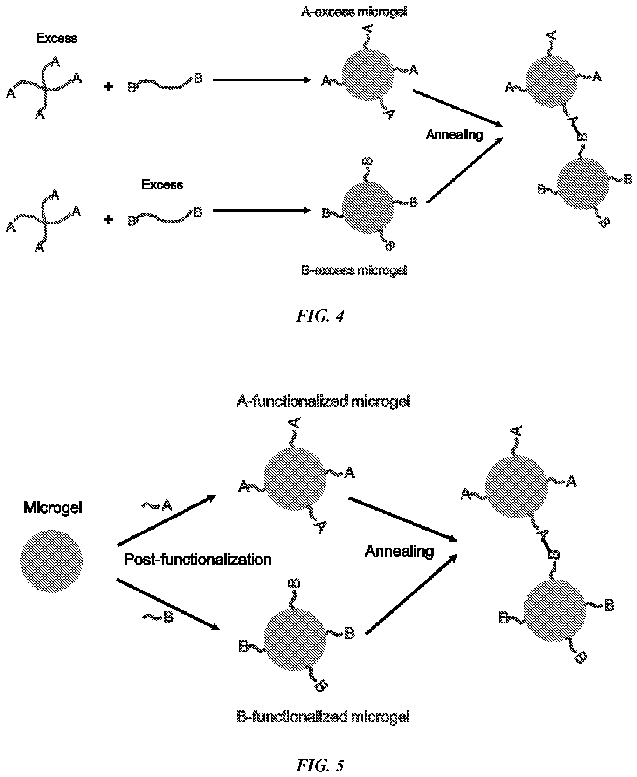

In examples not claimed , methods disclosed herein comprise synthesizing flowable microgel particles in the presence of at least one annealing component. This may be referred to as pre-functionalization of the flowable microgel particles. The annealing component may be part of the intracrosslinking component. The first annealing component may be part of the intracrosslinker of a microgel particle. The second annealing component may be part of the backbone polymer of a microgel particle. In examples not claimed, the methods comprise incorporating the first annealing component on to a first flowable microgel particle and incorporating the second annealing component on to a second flowable microgel particle. In examples not claimed , the methods comprise mixing the first annealing component with the second annealing component, such that there is an excess of the first annealing component *(e.g.,* ratio of first annealing component to second annealing component is greater than 1). In examples not claimed , the methods comprise mixing the first annealing component with the second annealing component, such that there is an excess of the second annealing component *(e.g.,* ratio of first annealing component to second annealing component is less thanl). The ratio of first annealing component to second annealing component may be about 0.1, about 0.2, about 0.3, about 0.5, about 0.8, about 1, about 1.2, about 1.5, about 1.8, or about 2. In examples not claimed , there is a difference between an amount of the first annealing component and the second annealing component. In examples not claimed , the difference is at least about 1%_{.} In examples not claimed, the difference is at least about 5%_{.} In examples not claimed , the difference is at least about 10%. In examples not claimed , the difference is at least about 20%. In examples not claimed, the difference is at least about 50%. In examples not claimed , the difference is at least about 100%. In examples not claimed, the methods further comprise annealing the first flowable microgel particle and the second flowable microgel particle via the first annealing component and second annealing component. In examples not claimed , annealing occurs *in situ.* In examples not claimed , annealing occurs *in vitro.* A schematic diagram is presented in **FIG. 4** to depict an example of pre-functionalization of flowable microgel particles. Examples of annealing components used for pre-functionalization include functional groups such as vinyl sulfone, thiol, amine, imidazole, aldehyde, ketone, hydroxyl, azide, alkyne, vinyl, alkene, maleimide, carboxyl, *N*-hydroxysuccinimide (NHS) ester, isocyanate, isothiocyanate, hydroxylamine, thione. By way of example, microgel particles containing an excess of vinyl sulfone groups can covalently react with microgel particles containing an excess of thiol groups by Michael addition to create a microporous scaffold. Additional examples of annealing components are described herein. In examples not claimed, pre-functionalization is desirable because it does not require further modification of particles after synthesis.

In examples not claimed , methods disclosed herein comprise synthesizing a flowable microgel particle and subsequently connecting an annealing component on to the microgel particle. This may be referred to as post-functionalization of the flowable microgel particles. A schematic diagram is presented in **FIG. 5** to depict an example of post-functionalization of flowable microgel particles. In examples not claimed , the methods comprise adding a first annealing component (A in **FIG. 5****)** to a first flowable microgel particle and adding a second annealing component (B in **FIG. 5****)** on to a second flowable microgel particle. Subsequently, the first flowable microgel particle *(e.g.,* first functionalized microparticle) is mixed with the second flowable microgel particle *(e.g.,* second functionalized microparticle) to anneal the first flowable microgel particle and the second flowable microgel particle via the first annealing component and second annealing component. Examples of connections that can be formed between a first annealing component and a second annealing component include a covalent bond *(e.g.,* amide, ester, C-C bond through Michael addition, carbamate, disulfide bond, oxime, thiourea, hydrazone, imine), a non-covalent bond through interaction such as *(e.g.,* electrostatic interactions, hydrogen bonding, cation-π, π-π stacking, metal-ligand binding, van der Waals interactions). Another example of an interaction that links or connects a first annealing component and a second annealing component is a non-covalent host-guest inclusion complex (driven by electrostatic interactions, hydrogen bonding, cation-π, π-π stacking, metal-ligand binding, or van der Waals interactions). In examples not claimed , the first flowable microgel particle and the second flowable microgel particle are mixed immediately before microgel application *(e.g.,* in a subject). In examples not claimed , the first flowable microgel particle and the second flowable microgel particle are mixed during microgel application *(e.g.,* from a multi-barrel syringe applied directly to a subject). In examples not claimed , annealing occurs *in situ.* In examples not claimed , the mixture anneals *in situ to* form a porous network. In examples not claimed , annealing occurs *in vitro.*

An example of post-functionalization, as described herein, is flowable microgel particles functionalized with DOPA reacting with flowable microgel particles functionalized with phenylboronic acid. Complexation of these flowable microgel particles form cyclic boronate esters, thereby creating a microporous scaffold. Another example is flowable microgel particles functionalized with beta-cyclodextrin interacting with flowable microgel particles functionalized with adamantane. Complexation of these flowable microgel particles form a host-guest inclusion complex, thereby creating a microporous scaffold. In some instances, post-functionalization is advantageous because it allows more functionality options than pre-functionalization. Post-functionalization may not be as simple and easy as pre-functionalization as it may require an extra step after particle synthesis. However, some annealing components cannot be added with the pre-functionalization method. For instance, some annealing components are unstable during particle synthesis and cannot be used to prefunctionalize, but can be added after particle synthesis (post-functionalization). So the post-functionalization may allow one to functionalize flowable microgel particles with a wider array of annealing components.

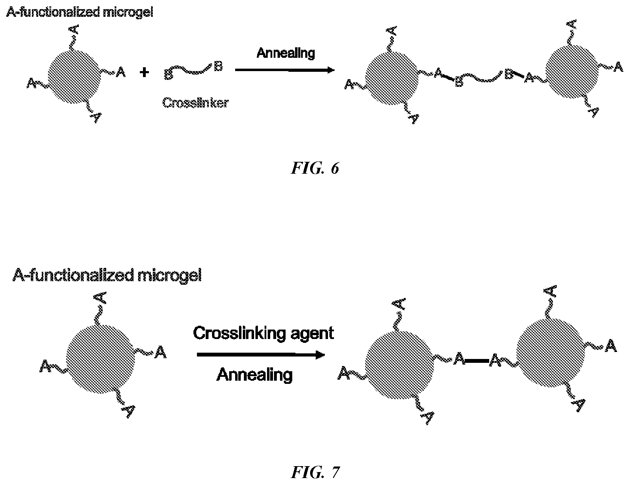

In examples not claimed , methods disclosed herein comprise annealing a first functionalized microgel particle to a second functionalized microgel particle using a crosslinker. At least one of the first functionalized microgel particle and the second functionalized microgel particle may be a pre-functionalized microgel particle, as described herein. At least one of the first functionalized microgel particle and the second functionalized microgel particle may be a postfunctionalized microgel particle, as described herein. In examples not claimed , the first functionalized microgel particle and the second functionalized microgel particle are the same. In some instances, the first functionalized microgel particle and the second functionalized microgel particle are different. A schematic diagram is presented in **FIG. 6** to depict an example of annealing flowable microgel particles with the use of a crosslinker. In examples not claimed, methods disclosed herein comprise crosslinking the first functionalized microgel particle to the second functionalized microgel particle, wherein the crosslinking comprises linking at least one of the first functionalized microgel particle and the second functionalized microgel particle with a crosslinker (B in **FIG. 6**). In examples not claimed, the methods comprise contacting at least one of the first functionalized microgel particle and the second functionalized microgel particle with a crosslinker after synthesizing the flowable microgel particles. In examples not claimed , the contacting occurs *in situ.* In examples not claimed, the contacting occurs *in situ* when the flowable microgel particles are being applied to tissue of a subject. The crosslinker may be functionalized with two or more functional groups. Examples of crosslinker functional groups are vinyl sulfone, thiol, amine, imidazole, aldehyde, ketone, hydroxyl, azide, alkyne, vinyl, alkene, maleimide, carboxyl, N-Hydroxysuccinimide (NHS) ester, isocyanate, isothiocyanate, hydroxylamine, and thione. The crosslinker can be homofunctional (same functional groups) or heterofunctional (different functional groups). Examples of crosslinking reactions using crosslinkers, include Michael addition, amide bond coupling, "click" chemistry (e.g. Diels-Alder cycloaddition, Huisgen 1,3-dipolar cycloaddition), reductive amination, carbamate linkage, ester linkage, thioether linkage, disulfide bond, hydrazone bond, oxime coupling, and thiourea coupling.

In examples not claimed, methods disclosed herein comprise annealing a first functionalized microgel particle to a second functionalized microgel particle, using a crosslinking agent. See, *e.g.,* **FIG.** 7. At least one of the first functionalized microgel particle and the second functionalized microgel particle may be a pre-functionalized microgel particle, as described herein. At least one of the first functionalized microgel particle and the second functionalized microgel particle may be a post-functionalized microgel particle, as described herein. In examples not claimed, the first functionalized microgel particle and the second functionalized microgel particle are the same. In examples not claimed, the first functionalized microgel particle and the second functionalized microgel particle are different. In examples not claimed, the crosslinking agent is added after synthesizing the functionalized microgel particles. In examples not claimed, the crosslinking agent is added during *in situ* application of the microgel particles to the tissue. In examples not claimed , the crosslinking agent is a reducing agent. Examples of reducing agents are dithiothreitol, dithioerythritol, L-glutathione, and tris (2-carboxyethyl) phosphine hydrochloride). In examples not claimed, the crosslinking agent is an oxidizing agent. The oxidizing agent may be a metal complexing agent. The oxidizing agent may be a catalyst. Examples of oxidizing agents are horseradish peroxidase (HRP), sodium periodate, and silver nitrate. In examples not claimed, the crosslinking agent induces self-crosslinking of the annealing components present on the flowable microgel particles. The resulting crosslinkage may comprise at least one of a covalent bond, a coordination complex, a hydrogen bonding, an electrostatic interaction, a cation-π interaction, a π-π stacking, and a van der Waals interaction. By way of example, DOPA-functionalized microgel particles may be crosslinked using silver nitrate as an oxidizing agent, wherein silver nitrate oxidizes the catechol of the DOPA moieties into reactive quinones that can further react with another DOPA group.

In examples not claimed, methods comprise *in situ* triggered release of a crosslinker. In examples not claimed, methods comprise *in situ* triggered release of an annealing agent. Functionalized microgel particles, as described herein, may be annealed using a crosslinker, annealing agent, or a crosslinking agent described herein that is released upon a trigger during *in situ* application of the flowable microgel particles to the tissue. Functionalized microgel particles, as described herein, may be annealed using a crosslinker, annealing agent or crosslinking agent described herein that is released upon a trigger after *in situ* application of the flowable microgel particles to the tissue. In examples not claimed, a crosslinker, annealing agent or crosslinking agent is entrapped in a nanoparticle which is embedded in a microgel particle during microparticle synthesis. In examples not claimed , the annealing agent, crosslinker or crosslinking agent is released by an internal trigger. examples of internal triggers are tissue mediated hydrolysis, enzyme mediated hydrolysis, hydrolysis not mediated by tissue or enzymes, enzymolysis, redox change (e.g. oxidative stress), pH change, and temperature change. In examples not claimed , the annealing agent, crosslinker or crosslinking agent is released by an external trigger. Examples of external triggers are temperature, light, electromagnetic field, and ultrasound.

Methods of producing microporous gel systems disclosed herein may comprise incorporating a therapeutic agent into a scaffold. Incorporating the therapeutic agent may comprise diffusing the therapeutic agent into a collection of flowable microgel particles. Incorporating the therapeutic agent may comprise attaching the therapeutic agent to the flowable microgel particles. The therapeutic agent may be attached to a flowable microgel particle via a covalent bond. The therapeutic agent may be attached to a flowable microgel particle via a non-covalent bond. Incorporating the therapeutic agent may comprise photo-caging the therapeutic agent to the microparticles.

Provided but not claimed are methods of producing a microporous gel system disclosed herein, comprising encapsulating a therapeutic agent in a nanoparticle, and mixing the therapeutic agent and the nanoparticle with flowable microgel particles. The nanoparticle and the therapeutic agent may be lyophilized. Methods may comprise dissolving the nanoparticle and the therapeutic agent *(e.g.,* in an aqueous buffer) prior to mixing the nanoparticle and the therapeutic agent with the flowable microgel particles.

### Methods of Treatment and Uses

Provided but not claimed are methods of treating a site of a medical device in a tissue of a subject comprising administering to the site: a collection of flowable microgel particles comprising a backbone polymer and an annealing component; an annealing agent that links the flowable microgel particles together via the annealing component to form a stabilized scaffold of microgel particles having interstitial spaces therein. The medical device may be any medical device disclosed herein. The medical device may be a surgical device, a medical implant or a biomaterial disclosed herein.

Provided but not claimed are methods of treating a cardiac arrhythmia comprising administering to a chest region of a subject in need thereof a medical device, wherein the medical device is a cardiac implantable electronic device; a collection of flowable microgel particles comprising a backbone polymer and an annealing component; and an annealing agent that links the flowable microgel particles together via the annealing component to form a stabilized scaffold of microgel particles having interstitial spaces therein.

Provided but not claimed are methods of treating a neurological condition, comprising administering to a spinal region or a brain region of a subject in need thereof, a medical device, wherein the medical device is a neural implantable electronic device; a collection of flowable microgel particles comprising a backbone polymer and an annealing component; and an annealing agent that links the flowable microgel particles together via the annealing component to form a stabilized scaffold of microgel particles having interstitial spaces therein.

The methods comprise administering the medical device to a tissue of the subject. The tissue may be skin. The tissue may be muscle. The tissue may be fascia. The tissue may be brain tissue. The tissue may be intestinal tissue. The tissue may be adipose tissue. The tissue may also be characterized as a tissue at a location of the subject. The location may a brain, a skull, a spine, an ear, an eye, a nasal sinus, a neck, a chest, an abdomen, a stomach, a shoulder, a hip, a pelvis, a leg, an arm, a knee, an elbow, a hand, a foot, a heart, an organ.

The methods may comprise administering the collection of flowable microgel particles to the site before the medical device contacts the site. The methods may comprise administering the collection of flowable microgel particles to the site after the medical device contacts the site. The methods may comprise co-administering the collection of flowable microgel particles and the medical device to the site. The methods may comprise administering the annealing agent to the site before administering the flowable microgel particles to the site. The methods may comprise administering the annealing agent to the site after administering the flowable microgel particles to the site. The methods may comprise co-administering the collection of flowable microgel particles and the annealing agent to the site. The collection of flowable microgel particles and the annealing agent may be coating the device or contained in and/or on the medical device before the medical device is implanted.

The methods may comprise administering a therapeutic agent disclosed herein, a backbone polymer disclosed herein, a degradable crosslinker disclosed herein, a cell adhesive peptide, or any combination thereof, to the site. The methods may comprise administering the therapeutic agent, the degradable crosslinker, the cell adhesive peptide, or any combination thereof, to the site after, before or concurrently with the medical device, flowable microgel particles or annealing agent. The methods may comprise applying the therapeutic agent, the degradable crosslinker, the cell adhesive peptide, or any combination thereof, to the medical device.

The methods may comprise applying a stimulus to the site, wherein the stimulus forms the stabilized scaffold. By way of example, the stimulus may be a chemical, an enzyme, an agent that alters the pH of the site or the microporous gel system, a redox stress, heat, cold, magnetic field, light, ultrasound, electrical field, radiation, and combinations thereof. Although the effects of the stimulus may last longer, the stimulus may be applied for about 1 second, about 2 seconds, about 3 seconds, about 4 seconds, about 5 seconds, about 10 seconds, about 30 seconds, about 1 minute, about 2 minutes, about 5 minutes, about 10 minutes, about 15 minutes, about 20 minutes, about 30 minutes, about 1 hour, about 2 hours, about 8 hours, about 12 hours, or about 1 day. The stimulus may be applied for 1 second, 2 seconds, 3 seconds, 4 seconds, 5 seconds, 10 seconds, 30 seconds, 1 minute, 2 minutes, 5 minutes, 10 minutes, 15 minutes, 20 minutes, 30 minutes, 1 hour, 2 hours, 8 hours, 12 hours, or 1 day. In some cases, the stimulus is applied for more than one day. The stimulus may be applied for about 1 second to about 1 day. The stimulus may be applied for about 5 seconds to about 1 day. The stimulus may be applied for about 10 seconds to about 1 day. The stimulus may be applied for about 30 seconds to about 1 day. The stimulus may be applied for about 1 minute to about 1 day. The stimulus may be applied for about 5 minutes to about 1 day. The stimulus may be applied for about 15 minutes to about 1 day. The stimulus may be applied for about 30 minutes to about 1 day. The stimulus may be applied for about 1 hour to about 1 day. The stimulus may be applied for 1 second to 1 day. The stimulus may be applied for 5 seconds to 1 day. The stimulus may be applied for 10 seconds to 1 day. The stimulus may be applied for 30 seconds to 1 day. The stimulus may be applied for 1 minute to 1 day. The stimulus may be applied for 5 minutes to 1 day. The stimulus may be applied for 15 minutes to 1 day. The stimulus may be applied for 30 minutes to 1 day. The stimulus may be applied for 1 hour to 1 day. The stimulus may be applied for about 1 second to about 12 hours. The stimulus may be applied for about 5 seconds to about 12 hours. The stimulus may be applied for about 10 seconds to about 12 hours. The stimulus may be applied for about 30 seconds to about 12 hours. The stimulus may be applied for about 1 minute to about 12 hours. The stimulus may be applied for about 5 minutes to about 12 hours. The stimulus may be applied for about 15 minutes to about 12 hours. The stimulus may be applied for about 30 minutes to about 12 hours. The stimulus may be applied for about 1 hour to about 12 hours. The stimulus may be applied for 1 second to 12 hours. The stimulus may be applied for 5 seconds to 12 hours. The stimulus may be applied for 10 seconds to 12 hours. The stimulus may be applied for 30 seconds to 12 hours. The stimulus may be applied for 1 minute to 12 hours. The stimulus may be applied for 5 minutes to 12 hours. The stimulus may be applied for 15 minutes to 12 hours. The stimulus may be applied for 30 minutes to 12 hours. The stimulus may be applied for 1 hour to 12 hours. The stimulus may be applied for about 1 second to about 1 hour. The stimulus may be applied for about 5 seconds to about 1 hour. The stimulus may be applied for about 10 seconds to about 1 hour. The stimulus may be applied for about 30 seconds to about 1 hour. The stimulus may be applied for about 1 minute to about 1 hour. The stimulus may be applied for about 5 minutes to about 1 hour. The stimulus may be applied for about 15 minutes to about 1 hour. The stimulus may be applied for about 30 minutes to about 1 hour. The stimulus may be applied for about 1 hour to about 1 hour. The stimulus may be applied for 1 second to 1 hour. The stimulus may be applied for 5 seconds to 1 hour. The stimulus may be applied for 10 seconds to 1 hour. The stimulus may be applied for 30 seconds to 1 hour. The stimulus may be applied for 1 minute to 1 hour. The stimulus may be applied for 5 minutes to 1 hour. The stimulus may be applied for 15 minutes to 1 hour. The stimulus may be applied for 30 minutes to 1 hour.

Methods of treatment and uses provided but not claimed may result in a tissue remodeling or cellular effect in the subject. In some instances, use of microporous gel system to microporous scaffold disclosed herein results in eliminating inflammation at a wound or surgical site. Elimination of inflammation may be evidenced by an absence of MNGC at the wound or surgical site. Elimination of inflammation may be evidenced by a similar number of MNGC at the wound or surgical site as compared to a non-wound site *(e.g.,* healthy, non-damaged tissue). Elimination of inflammation may be evidenced by a reduction or absence of neutrophils or macrophages at the wound or surgical site. Elimination of inflammation may be evidenced by a similar number of neutrophils or macrophages at the wound or surgical site as compared to a non-wound site *(e.g.,* healthy, non-damaged tissue). In examples not claimed , tissue of the subject integrates with the microporous scaffold. In examples not claimed, at least a portion of the microporous scaffold degrades *in situ.* In examples not claimed, at least about 10% of the microporous scaffold degrades *in situ.* In some instances, at least about 30% of the microporous scaffold degrades *in situ.* In examples not claimed, at least about 60% of the microporous scaffold degrades. In examples not claimed , at least about 90% of the microporous scaffold degrades *in situ.* In examples not claimed, the microporous scaffold completely degrades *in situ.* In examples not claimed, at least one of a wound site and a microporous scaffold is vascularized after administration of a gel disclosed herein. Vascularization may result in the presence of large vessels with intimal walls. Large vessels may be vessels, wherein at least a portion of the blood vessel has a diameter greater than about 10 µm. In some instances, the wound site develops a web-like dermal tissue, which indicates non-fibrous tissue formation. In some instances, the wound site does not develop fibrous tissue.

The extent of any tissue remodeling or cellular effect described herein, (*e.g.,* elimination of inflammation, integration of tissue, degradation of the microporous scaffold, and vascularization of the microporous scaffold), may occur within a time range from the time the gel was administered. The time range may depend upon the tissue to which the microporous scaffold is administered. The time range may be about 5 days to about 10 days. The time range maybe about 10 days to about 20 days. The time range may be about 20 days to about 30 days. The time range may be about 30 days to about 40 days. The time range may be about 40 days to about 50 days. The time range may be about 50 days to about 100 days.

Methods of administering a microporous gel system and device to a subject may result in microgel particle - substrate annealing, wherein a bond forms between a flowable microgel particle and a substrate (medical device or tissue of the subject). The bond can be covalent. The bond can be non-covalent. In examples not claimed, the bond forms between a flowable microgel particle and a device coating. In examples not claimed, the medical device is pre-coated with a functional group that is capable of binding to at least one flowable microgel particle of a collection of flowable microgel particles. In examples not claimed, the substrate binds to a first layer of flowable microgel particles, the latter of which binds to a second layer of microgel particles. The first layer of flowable microgel particles may comprise the same type of flowable microgel particles as the second layer of flowable microgel particles. The first layer of flowable microgel particles may comprise a different type of flowable microgel particles as the second layer of flowable microgel particles. In examples not claimed, microgel particle-substrate annealing occurs simultaneously with particle-particle annealing. In examples not claimed, microgel particle-substrate annealing occurs before particle-particle annealing. In examples not claimed, microgel particle-substrate annealing occurs after particle-particle annealing.

### Diseases and Conditions

Provided but not claimed are methods and systems for the treatment of a condition or disease in a subject, comprising administering any combination of microporous gel systems, medical devices and therapeutic agents disclosed herein. The condition or disease may be an acute condition or disease. By way of example, the acute condition or disease may be a dermal wound, a deep surgical wound, an amputation, or a stroke. The condition or disease may be a chronic condition or disease. By way of example, the chronic condition or disease may be a non-healing wound, heart arrhythmia, epilepsy, or osteoarthritis. The condition or disease may be a degenerative disease. By way of example, the degenerative disease may be a neurodegenerative disease *(e.g.,* Alzheimer's, Parkinson's or multiple sclerosis) or a cancer. The condition or disease may be a metabolic condition or disease *(e.g.,* diabetes). By way of example, the metabolic condition or disease may be diabetes or obesity. The condition or disease may be an orthopedic disorder *(e.g.,* musculoskeletal trauma, arthritis, fractures, infections, osteoporosis, ligament injuries).

Provided, but not claimed, are methods and systems for the treatment of a cardiovascular condition, a cardiovascular disease, a heart condition or a heart disease disclosed herein. A cardiovascular condition or cardiovascular disease is a condition or disease wherein vasculature of the subject is affected. The heart condition or heart disease may be a condition or disease that affects the function of the heart, such as the electrical function, pumping function or valve function, without affecting the health of the vasculature of the heart or cardiovascular system. The methods disclosed but not claimed provide for treatment of both heart and cardiovascular diseases and conditions, and combinations thereof. However, these terms may be used interchangeably herein, unless otherwise specified. Heart conditions include, atrial fibrillation, ventricular fibrillation, chronic heart failure, coronary artery disease, myocarditis, peripheral arterial occlusive disease, cardiomyopathy, pericarditis, myocarditis, endocarditis, a congenital heart defect, atherosclerosis, and combinations thereof. Heart conditions include cardiac arrhythmias. The cardiac arrhythmia may be acute. The cardiac arrhythmia may be chronic. The cardiac arrhythmia may be environmentally induced. The cardiac arrhythmia may be exercise induced. The cardiac arrhythmia may be caused by a genetic mutation. The cardiac arrhythmia may be caused by an infection.

Provided but not claimed are methods and systems for the treatment of a neurological disease or a neurological condition disclosed herein. The neurological condition may be chronic. The neurological condition may be acute. The neurological condition may be due to an injury. By way of example, the neurological disease or neurological condition may be Parkinson's Disease, Alzheimer's Disease, tremor, dystonia, chronic pain, major depression, obsessive compulsive disorder, schizophrenia, epilepsy, addictions, stroke, multiple sclerosis, traumatic brain injury, spinal cord injury, encephalitis, cerebral ischemia, or an intestinal condition. The neurological condition may be speech defect, hearing defect, paralysis, or partial-paralysis.

Any of the following examples that do not fall within the scope of the claims do not form part of the invention but are provided for illustrative purposes only

### EXAMPLES

### Example 1. Synthesis of flowable microgel particles

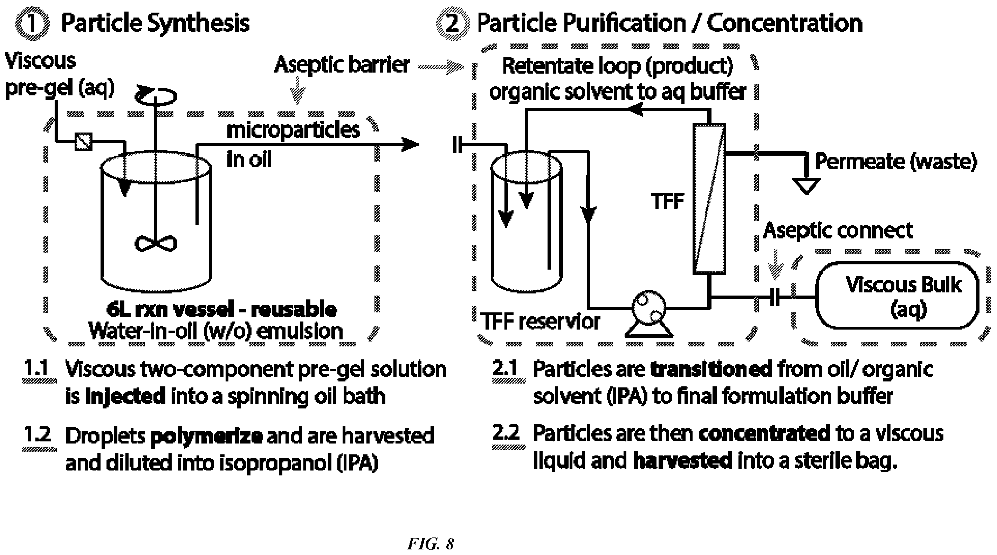

Flowable microgel particles were synthesized by a water-in-oil emulsion and purified by tangential flow filtration (TFF) *(see* **FIG. 8****).** The manufacturing process was performed aseptically. The reaction vessel, glassware, connectors, fittings, filters, tubing and TFF system were depyrogenated and then sterilized using a sterilization technique *(e.g.,* gamma radiation or moist heat (autoclave)). All the prepared solutions were filtered prior to the addition to the reaction vessel. Buffers and solvents used for purification were added to a sterile bag through a 0.2 µ 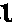 η filter.

*Oilphase preparation:* 7 L of light mineral oil (LMO) with 70 mL of span80 (1%v/v) was prepared. 5.7 L of the LMO+span80 mixture was added to a 6-L bioreactor vessel through a 0.2 µm filter and stirred with a 70-mm impeller rod at 800 rpm.

*PEG intracrosslinker solutionpreparation:* an aqueous solution containing 10%w/v 4-arm polyethylene glycol) vinyl sulfone (PEG-VS) (20 kDa), 500 µM K-peptide **(Ac-**FKGGERCG-NH2), 500 µM Q-peptide (Ac-NQEQVSPLGGERCG-NH2) and 1 mM RGD **(Ac-**RGDSPGERCG-NH2) was prepared in 300 mM triethanolamine (pH 7.75). The PEG solution was filtered using a 0.22 µm Stericup PES filter.

*Peptide Intracrosslinker solution preparation:* an 8 mM di-cysteine MMP- sensitive peptide (Ac-GCRDGPQGrvVGQDRCG-NH2) aqueous solution was prepared. The crosslinker solution was filtered using a 0.22 µm Stericup PES filter.

*Water-in -oil (wlo) emulsion:* 150 mL of the PEG solution was mixed 1:1 to 150 mL of the crosslinker solution. Immediately after mixing, the aqueous mixture (300 mL, 5%v/v w/o) was injected using a peristaltic pump (135 mL/min) to the stirring oil phase. After 2 h of stirring, the particles were allowed to settle down overnight and accumulated at the bottom of the reaction vessel. Because the particles are denser that LMO, they settled and accumulated at the bottom of the 6-L reaction vessel.

Approximately 80% (-4.8 L) of the oil phase was removed using a peristaltic pump and dip tubes of the vessel.

The particles were redispersed in 4.8 L of 95% isopropanol/5% water (referred to as 95% IPA solution) and stirred for at least 5 min at 450 rpm.

The particles were harvested in a 50 L sterile bag: at the same rate liquid is removed from the 6-L vessel, 95% IPA solution was added to the vessel to keep the volume constant (6 L) during particle harvest until 50 L of 95% IPA solution has been transferred through the 6-L vessel into the harvest bag.

### Example 2. Purification of Flowable Microgel Particles

Tangential flow filtration was used to purify and concentrate flowable microgel particles produced as described in Example 1.

The membrane used in this system was a Spectrum hollow fiber mPES membrane (P/N N02-E65U-07-N, pore rating = 0.65 µ 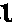 η, lumen ID = 0.75 mm, surface area 1,800 cm²).

The TFF system was gamma irradiated for sterilization.

The TFF system was plumbed to a lab stand with a Master Flex pump installed onto the retentate loop *{see* **FIG. 8****).** Spectrum Labs luer lock pressure transducers were placed on the filter inlet, outlet, and permeate in order to monitor the flows. The 50-L bag containing the harvested microparticles dispersed in 95% IPA solution was connected to the TFF inlet manifold.

The particles were transferred from the bag into the TFF reservoir (V = 3.5 L) and circulated through the retentate loop at 5 L/min (permeate valve close).

The particles were slowly concentrated (permeate valve open): the volume in the reservoir (3.5 L) was kept constant by pumping in microparticles as volume is removed through the permeate. The permeate flow was adjusted to target a filtrate flux rate between 200 and 300 LMH.

A gradient of solvents (from 95% isopropanol to 100% water and finally buffer) was used to purify the particles. All the solvents and buffers were stored in a sterile bag. A constant volume diafiltration was performed with 20 L of each of the following solutions: 95% IPA solution, 50% IPA solution, 100% pure water, and final formulation buffer (10 mM phosphate buffer + 100 mM NaCl + 5 µM Eosin Y, pH 7.4). The permeate flow was adjusted to target a filtrate flux rate between 200 and 300 LMH.

The particles were concentrated by TFF until the material reached the targeted concentration.

The particles were harvested in sterile bags and eventually concentrated further by centrifugation if necessary.

### Example 3. Wound Healing in Pigs Using Microporous Gel Systems

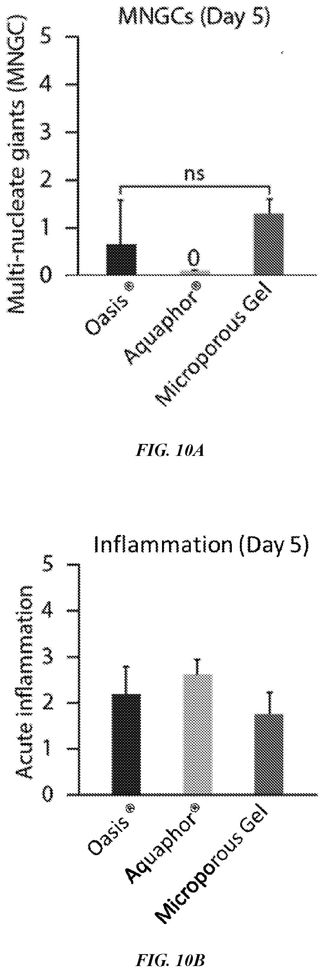

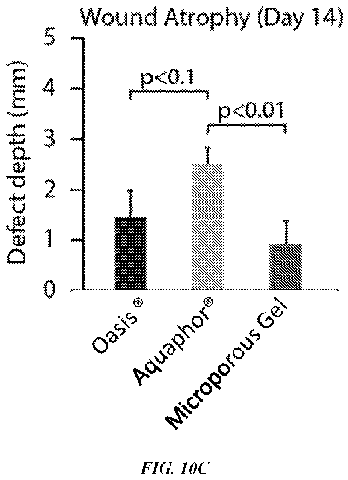

Wound beds in pigs were completely filled by a microporous gel that stabilized into a microporous scaffold. The microporous gel was produced as described in Example 1. Briefly, the microporous gel was made of the backbone polymer 4-ARM-PEG-VS + MMP-degradable peptide + RGD+K+Q peptides in final formulation buffer (PBS + 5 uM Eosin Y). The gel was annealed by light with eosin Y as an annealing agent. As controls, pigs with similar defects were treated with an Oasis^{®} SIS matrix and Aquaphor^{®}. Cross-sections of the wounds were examined after five days. Granulation tissue was stimulated in all test cases, as measured by tissue staining (data not shown, but available). Oasis^{®} SIS matrix and the microporous gel show similar, low acute multinucleated giant cell (MNGC) formation (while Aquaphor^{®} shows none), *see* **FIG. 10A****.** Acute inflammation was reduced in the wound beds receiving the microporous gel when compared to Oasis^{®} SIS matrix to Aquaphor^{®}, *see* **FIG. 10B****.** After 14 days of healing, wound atrophy was reduced by both the Oasis^{®} SIS matrix and the microporous scaffold, *see* **FIG IOC.** Cross-section histology showed the wound beds were completely filled by all the microporous gel 5 days after treating, while defects remain for both the Oasis^{®} SIS matrix and the Aquaphor^{®} treated wounds.

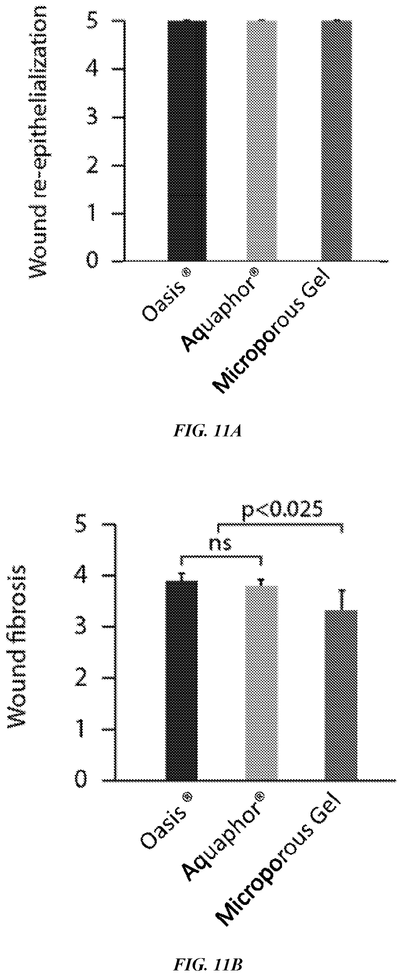

Wound re-epithelialization and tissue fibrosis were examined in tissue after 14 days of healing. Complete re-epithelialization was seen for all wounds treated with Oasis^{®} SIS matrix, Aquaphor^{®}, or the microporous gel, *see* **FIG. 11A****.** When examining the reformed tissue after 14 days, pathologic scoring indicated that tissue replacing the microporous scaffold exhibited less alignment in the collagen fiber bundles, and less dense bundling, indicative of tissue architecture different than that of fibrous scar tissue, as compared to the other cohorts (Oasis^{®} SIS matrix, Aquaphor^{®}). Quantification of fibrosis scoring is presented in **FIG. 11B****.**

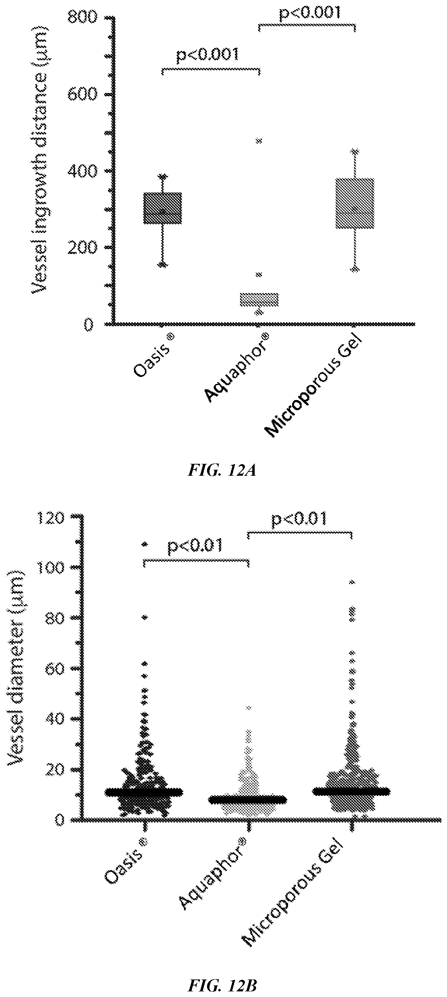

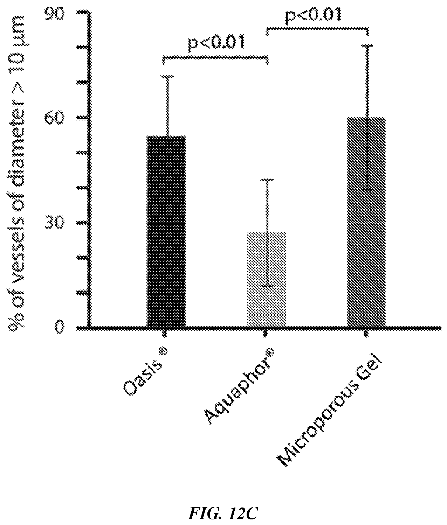

Augmentation of wound healing vascularization by the MAP gel was observed. Measurements of vessel ingrowth 5 days after healing showed that both Oasis^{®} SIS matrix and the microporous scaffold promoted increased depth of vascular penetration into the wound site (or scaffold), compared to Aquaphor^{®} standard care, (data not shown, but available). Vessel ingrowth quantification shows statistically significant augmentation of vascularization by Oasis^{®} SIS matrix and the microporous scaffold, *see* **FIG. 12A****.** High magnification images showed ingrowing vessels in the Oasis^{®} SIS matrix and microporous scaffold cohorts, (data not shown, but available). Both the Oasis^{®} SIS matrix and microporous scaffold microporous scaffold led to larger caliber vessel formation in healed tissue after 14 days, compared to Aquaphor^{®} treatment, (data not shown, but available). Aquaphor^{®} forms typical scar-like small vessels, averaging 5-10 µm in diameter (-capillary size). Both Oasis^{®} SIS matrix and the microporous scaffold promote larger vessel formation, *see* **FIG. 12B****,** and while these tissues also contain capillaries, the percentage of vessels larger than 10 µm in diameter significantly increases compared to Aquaphor^{®} treated wounds. *See* **FIG. 12C****.**

Both microporous gel injections and bilateral non-porous hydrogel injections (chemically identical but no microporous structure) were collected after 38 days *in vivo* and qualitatively assessed for tissue integration. The non-porous flowable hydrogel (Oasis^{®} SIS matrix) exhibited virtually no tissue ingrowth, with consistent MNGCs surrounding the material edge, typical of the Foreign Body Response. Microporous gel injections showed no detectable inflammation or MNGC presence around the injection periphery, almost complete tissue integration, and significant material degradation. The presence of large vessels with intimal walls, and web-like dermal tissue indicated non-fibrous tissue formation *de novo* within the injection site (data not shown, but available).

### Example 4. Administration of a Microporous Gel to a Site of a Cardiac Pacemaker

A physician performs surgery on a patient to place a pacemaker in the chest of the patient. The physician inserts the pacemaker in the left shoulder area where an incision is made below the collar bone creating a small pocket where the pacemaker battery pack and part of the leads are actually housed in the patient's body. The lead is fed into the heart through a large vein. Either: (i) after the pacemaker is inserted, a solution containing microgel particles and an annealing agent are applied to the incision site or (ii) a solution containing microgel particles and an annealing agent are applied to the incision site, followed by inserting the pacemaker. In either condition (i) or (ii), the solution flows around the pacemaker and fills any void between the pacemaker and surrounding tissue. The solution also contains an antibiotic, an analgesic, an anti-inflammatory agent and an anti-fibrotic agent. The surgical site is exposed to light and the microgel particles anneal to form a microporous scaffold. Alternatively, heteroannealing takes place, and light is unnecessary. The surgical site is sewn up. The patient heals quickly, experiences little pain or discomfort, and does not develop any infection at the surgical site. Eight years later, the physician creates another incision in the chest to replace the battery in the pacemaker. The physician notices that the pacemaker is integrated with the surrounding tissue better than a pacemaker in a patient that does not receive the microporous gel system. The physician also notices that there is less scar tissue around the pacemaker relative to a pacemaker in a patient that does not receive the microporous gel system. It is easier for the physician to remove or replace the pacemaker battery pack or leads, and the surgery time is reduced for this procedure - reducing the risk of infection during the procedure.

### Example 5. Spinal Cord Stimulation Implant for Spastic Cerebral Palsy

A surgical implant of electrodes for lateral cord stimulation is employed in patients with spastic cerebral palsy with the aim to improve tonus, motor function and speech. A unilateral hemilaminectomy is performed at C3-C4 level, starting from 4th cervical spinous process. A multicontact electrode is placed on the lateral surface of the spinal cord. The multicontact electrode is connected to a subcutaneously implanted pulse generator (IPG). In order to implant the IPG, a surgical void is created in the torsos of each patient. The IPG is placed within the surgical void. However, there is remaining space in the surgical void that is not filled by the IPG. A solution containing microgel particles and an annealing agent are applied to the incision site. The solution flows around the IPG and fills any void between the IPG and surrounding tissue. Less solution is used in patients where there is less remaining space as compared to more solution used in patients where there is more remaining space. In this way, the microporous gel system adapts the same device *(e.g.,* size, shape) to all patients. Alternatively, a solution containing microgel particles and an annealing agent are applied to the incision site prior to the placement of the IPG, and excess microgel particles are easily removed after IPG placement. The solution also contains an antibiotic, an analgesic, an anti-inflammatory agent and an anti-fibrotic agent. The surgical site is exposed to light and the microgel particles anneal to form a microporous scaffold. Alternatively, heteroannealing takes place, and light is unnecessary. The surgical site is sewn up. A post-operative evaluation is performed every 30 days for the next six months. The patients heal quickly, experience little pain or discomfort, and do not develop any infection at the surgical site. The devices work well, improving tonus, motor function and speech. If the device requires any re-intervention, the physician is able to access the device more easily than a patient that did not receive the microporous system, reducing surgical times and risks associated with that surgery.

### Example 6. Cardioverter-defibrillator Implant for Heart Arrhythmia

A cardioverter-defibrillator is implanted under the skin in the left upper chest of a patient with a ventricular arrhythmia. Either: (i) After the cardioverter-defibrillator is inserted, a solution containing microgel particles and an annealing agent are applied to the incision site or (ii) a solution containing microgel particles and an annealing agent are applied to the incision site, followed by inserting the cardioverter-defibrillator. In either condition (i) or (ii), the solution flows around the cardioverter-defibrillator and fills any void between the cardioverter-defibrillator and surrounding tissue. Risk of a venous obstruction such as upper extremity deep venous thrombosis and pulmonary embolism is historically high in patients receiving these devices. Thus, the solution contains an antithrombotic agent. The surgical site is exposed to light and the microgel particles anneal to form a microporous scaffold. Alternatively, heteroannealing takes place, and light is unnecessary. The surgical site is sewn up. The antithrombotic agent is released from the microporous scaffold over the next few weeks as the patient recovers. The patient does not develop or experience a vascular occlusion.

### Example 7. Testing Shelf-Life Stability of a Microporous Gel System

The shelf life and stability of the microporous scaffold is tested and validated using real-time and elevated temperature methods. Shelf life at 25°C, 50°C, and 100°C are determined by exposing the scaffold to these temperatures followed by undergoing the annealing process and measuring the increase in compressive modulus of the scaffold after annealing (compared to material that has not been exposed to elevated temperature and has been freshly prepared (no time passing since manufacture and measurement). Analytical methods (such as HPLC/DAD to measure peptide and light absorbing chemical components and GPC to measure polymeric chemical components) are also used to quantitate the shelf-life stability of the scaffold. After real-time and elevated temperature treatments, the scaffold is rinsed in aqueous buffer to extract degraded components, and that buffer is tested using the analytical methods to detect degradation products. Elevated temperatures (100°C) can be used to accelerate the stability process, where stability over short times at 100°C indicate stability over longer times at 25°C.

## Claims

1. A microporous gel system comprising:
a medical device for insertion into a surgical void of a subject;
i) flowable microgel particles, the flowable microgel particles having an average diameter of about 10 micrometers (µm) to 500 µm; wherein the flowable microgel particles comprise:
1. a backbone polymer; and
2. at least a first annealing component and a second annealing component, wherein the first annealing component and the second annealing component are linked together when exposed to a condition in the subject to form a stabilized microgel scaffold; and
ii) interstitial spaces occupying about 10% to about 40% of the total volume of the stabilized scaffold, wherein the stabilized microgel scaffold comprising a volume of microgel particles has a custom form that is determined by the surgical void and the medical device.

2. The system of claim 1, wherein the medical device is a cardiac implantable electronic device.

3. The system of claim 1, wherein the medical device is a neural implantable electronic device.

4. The system of any of claims 1 to 3, wherein at least one of the first annealing component and the second annealing component comprise a functional group selected from a vinyl sulfone, thiol, amine, imidazole, aldehyde, ketone, hydroxyl, azide, alkyne, vinyl, alkene, maleimide, carboxyl, N-hydroxysuccinimide (NHS) ester, isocyanate, isothiocyanate, hydroxylamine, thione, a catechol, a sialic acid, a boronic acid, a molecular cage, adamantane, biotin, and streptavidin.

5. The system of any preceding claim, wherein the first annealing component is covalently bonded to the second annealing component.

6. The system of any of claims 1 to 4, wherein the first annealing component is non-covalently bonded to the second annealing component, through a non-covalent bond selected from the group consisting of an electrostatic interaction, a hydrogen bond, a cation-π, π-π stack, a metal-ligand bond, a van der Waals interaction, and a non-covalent host-guest inclusion complex.

7. The system of any preceding claim, wherein the surgical void is closed.

8. The system of any preceding claim, wherein the stabilized microgel scaffold further comprises a therapeutic agent.

## Patentansprüche

1. Mikroporöses Gelsystem, umfassend:
eine medizinische Vorrichtung zur Insertion in einen chirurgischen Hohlraum eines Subjekts;
i) fließfähige Mikrogelpartikel, wobei die fließfähigen Mikrogelpartikel einen durchschnittlichen Durchmesser von etwa 10 Mikrometer (µm) bis 500 µm aufweisen; wobei die fließfähigen Mikrogelpartikel Folgendes umfassen:
1. ein Backbone-Polymer; und
2. mindestens eine erste Annealing-Komponente und eine zweite Annealing-Komponente, wobei die erste Annealing-Komponente und die zweite Annealing-Komponente miteinander verknüpft sind, wenn sie einem Zustand in dem Subjekt gegenüber ausgesetzt sind, um ein stabilisiertes Mikrogelgerüst zu bilden; und
ii) Zwischenräume, die etwa 10 % bis etwa 40 % des Gesamtvolumens des stabilisierten Gerüsts einnehmen, wobei das stabilisierte Mikrogelgerüst, das ein Volumen von Mikrogelpartikeln umfasst, eine benutzerdefinierte Form aufweist, die durch den chirurgischen Hohlraum und die medizinische Vorrichtung bestimmt wird.

2. System nach Anspruch 1, wobei die medizinische Vorrichtung eine kardiale implantierbare elektronische Vorrichtung ist.

3. System nach Anspruch 1, wobei die medizinische Vorrichtung eine neuronale implantierbare elektronische Vorrichtung ist.

4. System nach einem der Ansprüche 1 bis 3, wobei mindestens eine der ersten Annealing-Komponente und der zweiten Annealing-Komponente eine funktionelle Gruppe umfasst, die ausgewählt ist aus einem Vinylsulfon, Thiol, Amin, Imidazol, Aldehyd, Keton, Hydroxyl, Azid, Alkin, Vinyl, Alken, Maleimid, Carboxyl, N-Hydroxysuccinimid(NHS-)ester, Isocyanat, Isothiocyanat, Hydroxylamin, Thion, einem Katechol, einer Sialinsäure, einer Boronsäure, einem molekularen Käfig, Adamantan, Biotin und Streptavidin.

5. System nach einem vorhergehenden Anspruch, wobei die erste Annealing-Komponente kovalent an die zweite Annealing-Komponente gebunden ist.

6. System nach einem der Ansprüche 1 bis 4, wobei die erste Annealing-Komponente nichtkovalent an die zweite Annealing-Komponente durch eine nicht-kovalente Bindung gebunden ist, die ausgewählt ist aus der Gruppe bestehend aus einer elektrostatischen Interaktion, einer Wasserstoffbindung, einem Kation-π, π-π-Stapel, einer Metall-Ligand-Bindung, einer Van-der-Waals-Interaktion und einem nicht-kovalenten Wirt-Gast-Einschlusskomplex.

7. System nach einem vorhergehenden Anspruch, wobei der chirurgische Hohlraum geschlossen ist.

8. System nach einem vorhergehenden Anspruch, wobei das stabilisierte Mikrogelgerüst ferner ein Therapeutikum umfasst.

## Revendications

1. Système de gel microporeux comprenant :
un dispositif médical destiné à être inséré dans une cavité chirurgicale d'un sujet ;
i) des particules de microgel fluides, les particules de microgel fluides ayant un diamètre moyen d'environ 10 micromètres (µm) à 500 µm ; dans lequel les particules de microgel fluides comprennent :
1. un polymère de squelette ; et
2. au moins un premier composant d'assemblage et un second composant d'assemblage, dans lequel le premier composant d'assemblage et le second composant d'assemblage sont liés l'un à l'autre lorsqu'ils sont exposés à une condition présente chez le sujet pour former un échafaudage de microgel stabilisé ; et
ii) des espaces interstitiels occupant environ 10 % à environ 40 % du volume total de l'échafaudage stabilisé, dans lequel l'échafaudage de microgel stabilisé comprenant un volume de particules de microgel présente une forme personnalisée déterminée par la cavité chirurgicale et le dispositif médical.

2. Système de la revendication 1, dans lequel le dispositif médical est un dispositif électronique cardiaque implantable.

3. Système de la revendication 1, dans lequel le dispositif médical est un dispositif électronique implantable neuronal.

4. Système de l'une quelconque des revendications 1 à 3, dans lequel au moins l'un du premier composant d'assemblage et du second composant d'assemblage comprend un groupe fonctionnel choisi parmi une vinylsulfone, un thiol, une amine, un imidazole, un aldéhyde, une cétone, un hydroxyle, un azide, un alcyne, un vinyle, un alcène, un maléimide, un carboxyle, un ester de N-hydroxysuccinimide (NHS), un isocyanate, un isothiocyanate, une hydroxylamine, une thione, un catéchol, un acide sialique, un acide boronique, une cage moléculaire, un adamantane, une biotine et une streptavidine.

5. Système d'une quelconque revendication précédente, dans lequel le premier composant d'assemblage est lié de manière covalente au second composant d'assemblage.

6. Système de l'une quelconque des revendications 1 à 4, dans lequel le premier composant d'assemblage est lié de manière non covalente au second composant d'assemblage par une liaison non covalente choisie dans le groupe constitué par une interaction électrostatique, une liaison hydrogène, une cation-π, un empilement π-π, une liaison métal-ligand, une interaction de van der Waals et un complexe d'inclusion hôte-invité non covalent.

7. Système d'une quelconque revendication précédente, dans lequel la cavité chirurgicale est fermée.

8. Système d'une quelconque revendication précédente, dans lequel l'échafaudage de microgel stabilisé comprend en outre un agent thérapeutique.
